(19)

**Europäisches Patentamt**
**European Patent Office**
**Office européen des brevets**

(11) **EP 2 864 793 B1**

(12) **EUROPEAN PATENT SPECIFICATION**

(45) Date of publication and mention
of the grant of the patent:
**11.03.2020 Bulletin 2020/11**

(21) Application number: **13728209.1**

(22) Date of filing: **13.06.2013**

(51) Int Cl.:
***G01N 33/68*** (2006.01)

(86) International application number:
**PCT/EP2013/062231**

(87) International publication number:
**WO 2013/189822 (27.12.2013 Gazette 2013/52)**

(54) **METHODS FOR DETERMINING THE RISK OF MORTALITY IN PATIENTS WITH CARDIOVASCULAR DISEASE**

VERFAHREN ZUR BESTIMMUNG DES STERBLICHKEITSRISIKOS BEI PATIENTEN MIT KARDIOVASKULÄRER ERKRANKUNG

PROCÉDÉS POUR L'ÉVALUATION DU RISQUE DE MORTALITÉ D'UN PATIENT DE LA MALADIE CARDIOVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2012 EP 12172867**
**04.04.2013 EP 13162308**

(43) Date of publication of application:
**29.04.2015 Bulletin 2015/18**

(73) Proprietor: **LURIC Datenbank GbR**
**68165 Mannheim (DE)**

(72) Inventors:
• **GOLIASCH, Georg**
**A-1060 Wien (AT)**
• **NIESSNER, Alexander**
**A-1180 Wien (AT)**
• **KLEBER, Marcus E.**
**69412 Eberbach (DE)**
• **MÄRZ, Winfried**
**69493 Hirschberg/Leutershausen (DE)**

(74) Representative: **Nottrott, Stephanie**
**Patentanwälte**
**Isenbruck Bösl Hörschler PartG mbB**
**Prinzregentenstraße 68**
**81675 München (DE)**

(56) References cited:
• **J. H. IX ET AL: "Urinary Creatinine Excretion Rate and Mortality in Persons With Coronary Artery Disease: The Heart and Soul Study",** CIRCULATION, vol. 121, no. 11, 23 March 2010 (2010-03-23), pages 1295-1303, XP055038369, ISSN: 0009-7322, DOI: 10.1161/CIRCULATIONAHA.109.924266
• **P. STENVINKEL ET AL: "Emerging Biomarkers for Evaluating Cardiovascular Risk in the Chronic Kidney Disease Patient: How Do New Pieces Fit into the Uremic Puzzle?",** CLINICAL JOURNAL OF THE AMERICAN SOCIETY OF NEPHROLOGY, vol. 3, no. 2, 1 March 2008 (2008-03-01), pages 505-521, XP055038380, ISSN: 1555-9041, DOI: 10.2215/CJN.03670807
• **R. B. SCHNABEL ET AL: "Multiple marker approach to risk stratification in patients with stable coronary artery disease",** EUROPEAN HEART JOURNAL, vol. 31, no. 24, 2 December 2010 (2010-12-02), pages 3024-3031, XP055038381, ISSN: 0195-668X, DOI: 10.1093/eurheartj/ehq322
• **G. GOLIASCH ET AL: "Routinely available biomarkers improve prediction of long-term mortality in stable coronary artery disease: the Vienna and Ludwigshafen Coronary Artery Disease (VILCAD) risk score",** EUROPEAN HEART JOURNAL, vol. 33, no. 18, 28 June 2012 (2012-06-28), pages 2282-2289, XP055068399, ISSN: 0195-668X, DOI: 10.1093/eurheartj/ehs164

**(Cont. next page)**

EP 2 864 793 B1

- G. N. THOMAS ET AL: "Vitamin D Levels Predict All-Cause and Cardiovascular Disease Mortality in Subjects With the Metabolic Syndrome: The Ludwigshafen Risk and Cardiovascular Health (LURIC) study", DIABETES CARE, vol. 35, no. 5, 1 May 2012 (2012-05-01), pages 1158-1164, XP055068415, ISSN: 0149-5992, DOI: 10.2337/dc11-1714

**Description**

The present invention relates to the field of laboratory diagnostics. Specifically, methods for determining the risk of mortality in a patient with cardiovascular disease and to reduce the patient's risk of mortality according to the claims by providing treatments of secondary prevention to the patient are disclosed.

An aim of modern medicine is to provide personalized or individualized treatment regimens. Those are treatment regimens which take into account a patient's individual needs or risks. Individualized treatment regimens offer benefits for the individual patient as well as for society as a whole. For the individual patient personalized treatment avoids excessive therapy while ensuring that necessary measures are taken. As every therapy may cause undesired harmful side effects, the avoidance of unnecessary therapies saves the patient from potentially harmful side effects. On the other hand, the identification of patients with special needs ensures that these individuals receive the appropriate treatment. For the health system as a whole the avoidance of unnecessary therapies allows for a more economic use of resources.

Individualized treatment regimens require appropriate diagnostic tools in order to separate those patients who benefit from certain therapeutic measures from the patients who do not. Therefore, the development of individualized treatment regimens critically depends on the development of novel diagnostic tools and procedures. Because the prevention of future disease is often more effective than the therapy of already existing disease, diagnostic tools and methods for risk stratification with respect to future diseases are especially desirable.

Cardiovascular diseases are amongst the major causes of death in industrialized countries. Mortality from cardiovascular diseases substantially declined in Westernized countries during the past five decades (Ford, ES et al., 2007, N. Engl. J. Med. 356: 2388-2398). However, despite highly effective measures to control conventional risk factors the incidence of cardiovascular events remains high. This highlights the need to identify independent non-conventional risk factors for cardiovascular events. Therefore, there is always a need for improved methods of risk prediction in cardiovascular medicine.

Ix et al. (Circulation 121: 1295-1303, 2010) disclose the association of the creatinine excretion rate with mortality in persons with coronary artery disease. Stenvinkel et al. (Clin J Am Soc Nephrol 3: 505-521, 2008) disclose emerging biomarkers for evaluating the cardiovascular risk in patients with chronic kidney disease. Schnabel et al. (European Heart Journal 31: 3024-3031, 2010 (21)) disclose a multiple marker approach to risk stratification in patients with coronary artery disease. Thomas et al. (Diabetes Care 35: 1158-1164, 2012) disclose that Vitamin D levels predict all-cause and cardiovascular disease mortality in subjects with metabolic syndrome.

The invention is characterized by the subject-matter of the claims.

Moreover, disclosed, but not comprised by the present invention, is a method for determining the risk of mortality in a patient with a cardiovascular disease, comprising the steps of

a) analysing a set of biological parameters obtained from the patient, wherein the set of biological parameters comprises at least the following variables: the patient's age, an indicator of myocardial performance, an indicator of renal performance, the patient's heart rate, an indicator of blood glucose metabolism,

b) setting the biological parameter into correlation, thereby providing a mortality risk score, wherein the risk score is indicative for mortality of the patient within the next years, and wherein the risk score is indicative for a treatment of secondary prevention of the patient to reduce the patient's risk of mortality due to the cardiovascular disease.

Also, disclosed, but not comprised by the present invention, is a method for determining the risk of mortality in a patient with a cardiovascular disease, comprising the steps of

a) analysing a set of biological parameters obtained from the patient, wherein the set of biological parameters comprises the following variables: the patient's gender, the patient's age, at least one indicator of myocardial function or myocardial performance, at least two indicators of renal function or renal performance, the patient's heart rate, at least one indicator of blood glucose metabolism, and at least one indicator of blood calcium and/or phosphate metabolism,

b) setting the biological parameter into correlation, thereby providing a mortality risk score, wherein the risk score is indicative for mortality of the patient within the next years, and wherein the risk score is indicative for a treatment of secondary prevention of the patient to reduce the patient's risk of mortality due to the cardiovascular disease.

The term "determining the risk of mortality" as used herein refers to assessing the probability according to which a subject will die within a certain time window, i.e. the predictive window. In accordance with the present invention, the predictive window is preferably within 3 years, 5 years, 8 years, or 10 years or more after determination of the risk of mortality. However, as will be understood by those skilled in the art, such an assessment is usually not intended to be correct for 100 % of the subjects to be investigated. The term, however, requires that prediction can be made for a statistically significant portion of subjects in a proper and correct manner. Whether a portion is statistically significant can be determined without further ado by the person skilled in the art using various well known statistic evaluation tools, e.g., the Mann-Whitney-U test, Cox proportional hazard regression analysis, Pearson correlation and Harrell's C-statistic. Details are, for example, found in Dowdy and Wearden, Statistics for Research, John Wiley & Sons, New York 1983. Continuous data are presented as median and interquartile range, discrete data as counts and percentages. Continuous variables preferably undergo log transformation before entering analysis. Preferred confidence intervals are at least 95 %, at least 97 %, at least 98 % or at least 99 %. The p-values are, preferably, 0.005, or 0.0001. The discriminatory power of the designed risk score is assessed using Harrell's C-statistic. The improvement of individual risk prediction is preferably examined by net reclassification improvement as described by Pencina at al. (13). Preferably, the probability envisaged by the present invention allows that the prediction of an increased, normal or decreased risk will be correct for at least 60 %, at least 70 %, at least 80 %, or at least 90 %, and preferably of at least 95 % of the subjects of a given cohort or population. Determining the risk of mortality preferably relates to determining whether or not there is an increased risk of mortality compared to the average risk of mortality in a population of subjects rather than giving a precise probability for the said risk.

The method for determining the risk of mortality in a patient with a cardiovascular disease is an in vitro method. Moreover, it may comprise steps in addition to those explicitly mentioned. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

The risk of mortality to be determined is due to cardiovascular mortality. Cardiovascular mortality refers to all cases of death that are related to cardiovascular diseases. The term "cardiovascular disease" as used herein generally refers to a class of diseases that involve the heart or blood vessels. Cardiovascular disease further refers to any disease that affects the cardiovascular system (as described, for example, in the International Statistical Classification of Diseases and Related Health Problems 10th Revision (ICD10), ICD-10 Chapter IX: Diseases of the circulatory system). The causes of cardiovascular disease are diverse but atherosclerosis and/or hypertension are the most common. Death due to cardiovascular diseases may comprise myocardial infarction, death from congestive heart failure, death from stroke, death following coronary artery bypass graft surgery or death following percutaneous coronary intervention.

The term "patient" as used herein generally refers to a mammal, preferably to a human. The patient suffers from a cardiovascular disease, wherein the cardiovascular disease is a stable coronary artery disease, and may or may not suffer from diseases that increase the risk of death from cardiovascular causes. Said diseases preferably include, but are not limited to, arterial hypertension, renal failure, type 1 diabetes, type 2 diabetes and cardiovascular diseases such as, for example, cerebrovascular disease, stroke or peripheral vascular disease.

The term "mortality risk score" as used herein generally means an indicator for the risk of mortality with respect to a certain period of time. In particular, the mortality risk score provides an estimate of how long the patient is expected to survive a certain period of time, or alternatively, of how likely it is that the patient will die within this time window. Therefore, in the context of the present invention, the mortality risk score can either be seen as an indicator of a particular survival or mortality rate.

The term "biological parameter" as used herein generally includes all kind of gene expression products which are differentially expressed, i.e. up regulated or down regulated in presence or absence of a certain condition, disease, or complication. Usually, a biological parameter is also referred to herein as a biomarker. According to the present invention, a biological parameter may also refer to the patient's age, the patient's gender and/or the patient's heart rate. Equally preferred is that a biological parameter may be in form of a nucleic acid (including mRNA), a protein, a peptide, or a small molecule compound, preferably in form of its concentration or amount. The amount of a suitable biological parameter can indicate the presence or absence of the condition, disease, or complication, and thus allow diagnosis. For example, biological parameters reflecting inflammation, lipid metabolism, renal function and/or cardiovascular function are well known in the art including their analysis by means of commercially available routine assay systems and antibodies (see, for example, Schnabel et al., 2010. Eur Heart J, 31: 3024-3031).

In the method for determining the risk of mortality in a patient with a cardiovascular disease, as "indicator of myocardial performance" and/or "indicator of myocardial function" any biological parameter which provides information about the medical condition of the patient's heart can be used. An indicator of myocardial function or performance may mean a biological parameter that provides information about the cardiovascular function. Examples of biological parameters which indicate the patient's myocardial performance include, but are not limited to, copeptin, C-terminal-pro-endothelin-1, mid-regional-pro-adrenomedullin (MR-proADM), mid-regional-pro-atrial natriuretic peptide (MR-proANP), and N-terminal-pro-B-type natriuretic peptide (NT-proBNP). An indicator of myocardial performance may also refer to the patient's left ventricular ejection fraction (LVEF) which is a measurement of the percentage of blood leaving the patient's heart each time it contracts. In particular, the term "ejection fraction" refers to the percentage of blood that is pumped out of a filled ventricle with each heartbeat. Because the left ventricle is the heart's main pumping chamber, ejection fraction is usually measured only in the left ventricle (LV). A normal left ventricular ejection fraction is considered to be in the range of 55 to 70 %. Under certain circumstances, in particular in patients with cardiovascular diseases, the ejection fraction may decrease.

However, in the context of the present invention, the indicator of myocardial function or myocardial performance is defined by the patient's left ventricular ejection fraction (LVEF) in % of volume per volume. Techniques for measuring the patient's left ventricular ejection fraction (LVEF) are well known in the art and include, for example, echocardiogram, cardiac catheterization, magnetic resonance imaging (MRI), computerized tomography (CT), or nuclear medicine scan. In the context of the present invention, the left ventricular ejection fraction (LVEF) was categorized into normal ($\geq$ 55 %), mildly reduced (45-54 %), moderately reduced (30-44 %), and severely reduced (< 30 %). A teaching of how to measure and determine a left ventricular ejection fraction (LVEF) according to the invention is further illustrated by the Examples of the present application.

In the method for determining the risk of mortality in a patient with a cardiovascular disease, as alternative indicator of myocardial performance and/or the indicator of myocardial function, the patient's blood concentration of natriuretic peptides can be used. The term "natriuretic peptides" generally refers to all known atrial natriuretic peptides (ANP) and brain natriuretic peptides (BNP) described in the art (Bonow 1996, Circulation 93, 1946), including, for example, pre-proANP, proANP, NT-proANP, ANP and pre-proBNP, proBNP, NT-proBNP, and BNP. The pre-pro peptide (134 amino acids in the case of pre-proBNP) comprises a short signal peptide, which is enzymatically cleaved releasing the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Natriuretic peptides may be NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NT-proBNP is 120 min longer than that of BNP, which is 20 min (Smith 2000, J Endocrinol 167, 239). Preanalytics are more robust with NT-proBNP allowing easy transportation of the sample to a central laboratory (Mueller 2004, Clin Chem Lab Med 42, 942). Blood samples can be stored at room temperature for several days or may be mailed or shipped without recovery loss. In contrast, storage of BNP for 48 hours at room temperature or at 4° Celsius leads to a concentration loss of at least 20 % (Wu 2004, Clin Chem 50, 867). Therefore, depending on the time-course or properties of interest, either measurement of the active or the inactive forms of the natriuretic peptide can be advantageous. The natriuretic peptides may be NT-proBNP or variants thereof. The human NT-proBNP is a polypeptide comprising, preferably, 76 amino acids in length corresponding to the N-terminal portion of the human NT-proBNP molecule. A further natriuretic peptide may be the mid-regional-pro-atrial natriuretic peptide (MR-proANP). Methods for measurement natriuretic peptides have been described (Ala-Kopsala 2004, Clin Chem 50, 1576), and the concentration of the natriuretic peptides is preferably calculated in pg/ml, but may also be indicated by any other suitable concentration format, such as, for example, pmol/dl.

However, in the context of the present invention, the at least one indicator of myocardial function or myocardial performance is defined by the patient's left ventricular ejection fraction (LVEF) in % of volume per volume, or by the patient's blood concentration of N-terminal pro-B-type natriuretic peptide (NT-proBNP).

In the method for determining the risk of mortality in a patient with a cardiovascular disease, as "indicator of renal performance" and/or "indicator of renal function" any biological parameter which provides information about the medical condition of the patient's kidney(s) can be used. An indicator of renal function or performance may be a biological parameter or a biomarker that provides information about the renal function. Examples of biological parameters include, but are not limited to, cystatin C, serum creatinine, and renin (Schnabel et al., 2010. Eur Heart J; 31: 2024-3031).

However, in the context of the present invention, the indicator of renal performance is defined by the patient's blood concentration of serum creatinine. Serum creatinine is the most commonly used indicator of renal function. The concentration of serum creatinine can be measured in a blood sample by standard methods, such as the Jaffe routine method. The concentration of serum creatinine is preferably calculated in mg/dl, but may be indicated by any other suitable concentration format, such as, for example, pmol/dl.

However, in the context of the present invention, the at least two indicators of renal performance or renal function are defined by the patient's blood concentration of cystatin C and renin. Cystatin C, also referred to as cystatin 3 (and formerly known as gamma trace, post-gamma-globulin or neuroendocrine basic polypeptide) is a protein encoded by the CST3 gene. Renin is an enzyme produced by the kidney. Cystatin C and renin are routinely used biomarkers for evaluating the kidney function and/or performance of a patient. The concentration of cystatin C in serum can be defined by standard methods known in the art and is preferably measured, calculated and indicated in mg/L. The same applies for the concentration of renin in a patient's blood sample, the concentration of which is preferably indicated in pg/mL, $\mu$IU/mL, or equivalently in mIU/L, or U/L.

In the method for determining the risk of mortality in a patient with a cardiovascular disease, as "indicator of blood glucose metabolism" any biological parameter which provides information about the various biochemical processes responsible for the formation, breakdown and interconversion of carbohydrates in a living organism, including, but not limited to, the maintenance of steady levels of glucose in the body, can be used. An indicator of blood glucose metabolism may be a biological parameter that provides information about the average plasma glucose concentration over prolonged periods of time. Examples include, but are not limited to, the biological parameter of glycated haemoglobin Hbalc (also referred to as haemoglobin A1c, HbA1c, A1C, Hb1c, or HbA1c). The concentration of the glycated haemoglobin HbAlc is commonly measured in percentage (%) with respect to the total amount of haemoglobin. In general, the reference range found in healthy persons is about 20-40 mmol/mol (4% - 5.9 %). The 2010 American Diabetes Association Standards of Medical Care in Diabetes added the A1c $\geq$ 48 mmol/mol ($\geq$ 6.5 %) as a criterion for the diagnosis of diabetes.

However, in the context of the present invention, the or the at least one indicator of blood glucose metabolism is defined by the patient's blood concentration of glycated haemoglobin HbAlc in % of total haemoglobin.

The term "blood sample" as referred to herein preferably refers to a sample of blood which has been obtained from the patient by standard routine practice. In the context of the present invention, however, it is also included that the biological parameter(s) of interest is/are determined by the use of any other sample of the patient as input material, such as, for example, a sample of a body fluid, a sample of separated cells or a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which produce the marker referred to herein.

The patient's gender may be male or female.

In the method for determining the risk of mortality in a patient with a cardiovascular disease, as "indicator of blood calcium and/or phosphate metabolism" any biological parameter which is indicative for or related to the concentration or the level of calcium and/or phosphate, preferably calcium and/or phosphate ions, in the patient's blood, for example in the patient's blood serum, can be used. Examples of an indicator of blood calcium and/or phosphate metabolism include, but are not limited to, vitamin D (also known as cholecalciferol or calciol), for example vitamin $D_3$, for example 25(OH) vitamin $D_3$. Included are also any kind of metabolism substances related to the vitamin D pathway.

However, in the context of the present invention, the at least one indicator of blood calcium metabolism is defined by the patient's blood concentration of 25(OH)-vitamin $D_3$,

Disclosed, but not comprised by the present invention, is a method for determining the risk of mortality in a patient with a cardiovascular disease, comprising the steps of

a) analysing a set of biological parameters obtained from the patient, wherein the set of biological parameters comprises at least the following variables: the patient's age, an indicator of myocardial performance, an indicator of renal performance, the patient's heart rate, an indicator of blood glucose metabolism,

b) setting the biological parameter into correlation, thereby providing a mortality risk score, wherein the risk score is indicative for mortality of the patient within the next years, and wherein the risk score is indicative for a treatment of secondary prevention of the patient to reduce the patient's risk of mortality due to the cardiovascular disease, wherein

i) the indicator of myocardial performance is defined by the patient's left ventricular ejection fraction in % of volume per volume, or by the patient's blood concentration of natriuretic peptides,

ii) the indicator of renal performance is defined by the patient's blood concentration of serum creatinine, or by the patient's blood concentration of cystatin C,

iii) the indicator of blood glucose metabolism is defined by the patient's blood concentration of glycated haemoglobin HbAlc in % of total haemoglobin.

Disclosed, but not comprised by the present invention, is a method for determining the risk of mortality in a patient with cardiovascular disease, comprising the steps of

a) analysing a set of biological parameters obtained from the patient, wherein the set of biological parameters comprises the following variables: the patient's gender, the patient's age, at least one indicator of myocardial function or myocardial performance, at least two indicators of renal function or renal performance, the patient's heart rate, at least one indicator of blood glucose metabolism, and at least one indicator of blood calcium and/or phosphate metabolism,

b) setting the biological parameter into correlation, thereby providing a mortality risk score, wherein the risk score is indicative for mortality of the patient within the next years, and wherein the risk score is indicative for a treatment of secondary prevention of the patient to reduce the patient's risk of mortality due to the cardiovascular disease, wherein

i) the at least one indicator of myocardial function or myocardial performance is defined by the patient's left ventricular ejection fraction (LVEF) in % of volume per volume and/or by the patient's blood concentration of one or more natriuretic peptide(s), preferably by the patient's blood concentration of one or more natriuretic peptide(s), more preferably by the patient's blood concentration of N-terminal pro-B-type natriuretic peptide (NT-proBNP),

ii) the at least two indicators of renal function or renal performance are defined by the patient's blood concentration of renin and cystatin C,

iii) the at least one indicator of blood glucose metabolism is defined by the patient's blood concentration of glycated haemoglobin HbA1c in % of total haemoglobin,

iv) the at least one indicator of blood calcium metabolism is defined by the patient's blood concentration of vitamin $D_3$, preferably by the patient's blood concentration of 25(OH) vitamin $D_3$.

In the method for determining the risk of mortality in a patient with a cardiovascular disease, the mortality risk score can be a simplified risk score $X_1$ and the biological parameter are set into correlation by a sum of at least five quantities, the sum thereby providing the simplified risk score $X_1$,
wherein a first quantity is equal to 2 if the patient's age is equal to or more than 75 years and otherwise equal to 0,
wherein a second quantity is equal to 1 if the left ventricular ejection fraction is moderately reduced, equal to 2 if the left ventricular ejection fraction is severely reduced and otherwise equal to 0, wherein a moderately reduced left ventricular ejection fraction is defined by a value of 30 - 44 in % of volume per volume, and wherein a severely reduced left ventricular ejection fraction is defined by a value of below 30 in % of volume per volume,
wherein a third quantity is equal to 1 if the concentration of Hba1c is equal to or more than 6.5 % for patients with known diabetes and otherwise equal to 0,
wherein a fourth quantity is equal to 1 if the serum creatinine concentration is equal to or more than 1.3 mg/dl and otherwise equal to 0, and
wherein a fifth quantity is equal to 1 if the patient's heart rate is equal to or more than 75 beats per minute and otherwise equal to 0.

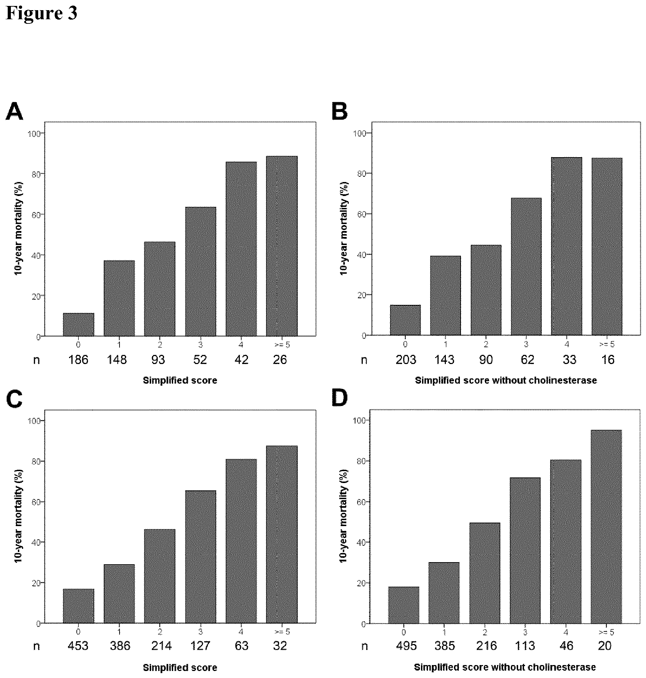

The simplified risk score $X_1$ and/or $X_{1a}$ has been designed for optimal clinical use and its calculation is exemplified in detail by the Examples of the present application, in particular by the results of Figure 3. In the context of the present invention, it has surprisingly been found that the simplified risk score $X_1$ and/or $X_{1a}$ has a strong predictive value for 10-year mortality with a C-statistic of 0.74 in the deviation cohort and of 0.7 in the validation cohort (both P< 0.001 for comparison with the conventional risk score).

[0032]    , The above set of biological parameters in the above method for determining the risk of mortality in a patient with cardiovascular disease, not comprised by the present invention, further comprises a variable in form of an indicator of liver performance. That is, the above method for determining the risk of mortality in a patient with a cardiovascular disease comprises the steps of

a) analyzing a set of biological parameters obtained from the patient, wherein the set of biological parameters comprises at least the following variables: the patient's age, an indicator of myocardial performance, an indicator of renal performance, the patient's heart rate, an indicator of blood glucose metabolism, and an indicator of liver performance,

b) setting the biological parameter into correlation, thereby providing a mortality risk score, wherein the risk score is indicative for mortality of the patient within the next years, and wherein the risk score is indicative for a treatment of secondary prevention of the patient to reduce the patient's risk of mortality due to the cardiovascular disease.

In the method for determining the risk of mortality in a patient with a cardiovascular disease, as the "indicator of liver performance" any biological parameter which provides information about the function of the patient's liver can be used. Information of liver performance can, for example, be obtained by carrying out clinical biochemistry laboratory blood assays designed to give information about the patient's liver function. The analysed biological parameters can include, for example, PT/INR, aPTT, albumin, bilirubin (direct and indirect) and others such as serum cholinesterase.

These tests are commercially available and standard routine to the person skilled in the art.

However, in the context of the present invention, the indicator of liver performance is defined by the patient's blood concentration of serum cholinesterase. Tests for determining the concentration of serum cholinesterase in a blood sample are standard routine in the art and may include the analysis of acetylcholinesterase and pseudocholinesterase levels. Typically, normal pseudocholinesterase values range between 8 and 18 units per milliliter (U/mL). Preferably, the concentration of serum cholinesterase according to the methods known in the art is calculated and indicated in Units/ml.

It has surprisingly been found that the calculation of a simplified risk score with or without the biological parameter of serum cholinesterase showed a similar predictive value in the derivation and validation cohorts and a comparable discrimination of mortality, as, for example, exemplified in Figure 3 of the present invention. However, it has also been found that low concentrations of serum cholinesterase have a strong inverse effect on mortality with a Hazard ratio of 0.57 per 1-SD increase in the derivation cohort and of 0.72 in the validation cohort, as exemplified by the Examples including Table 1 of the present invention.

Therefore, the biological parameters may be set into correlation by a sum of at least six quantities, the sum thereby providing the simplified risk score $X_{1a}$, wherein the first to the fifth quantities are defined as above and a sixth quantity is set to 1 if the concentration of serum cholinesterase is equal to or less than 4.4 Units/ml and otherwise equal to 0.

It has further surprisingly been found that the combination of at least eight, and preferably nine, routinely available biomarker which can be easily measured with a single blood draw resulted in an even improved biomarker score, and thereby mortality risk score, achieving a significantly improved discriminatory power with a C-static of 0.78 and a net reclassification index (NRI) of 14.9 % (P<0.001).

Hence, in an alternative aspect, the biological parameter are set into correlation by a sum of at least eight quantities, the sum thereby providing the simplified risk score $X_{1\#}$,

wherein a first quantity is equal to 2 if the patient's age is equal to or more than 75 years and otherwise equal to 0,

wherein a second quantity is equal to 1 if the concentration of N-terminal pro-B-type natriuretic peptide (NT-proBNP) is equal to or more than 400 ng/L, equal to 2 if the concentration of one or more natriuretic peptide(s) is equal to or more than 2000 ng/L and otherwise equal to 0,

wherein a third quantity is equal to 1 if the concentration of glycated haemoglobin HbAlc is equal to or more than 6.5 % for patients with known diabetes and otherwise equal to 0,

wherein a fourth quantity is equal to 1 if the concentration of renin is equal to or more than 50 pg/mL and otherwise equal to 0,

wherein a fifth quantity is equal to 1 if the concentration of cystatin C is equal to or more than 1.2 mg/L or otherwise equal to 0,

wherein a sixth quantity is equal to 1 if the concentration of 25(OH) vitamin $D_3$ is equal to or less than 10 ng/L and otherwise equal to 0,

wherein a seventh quantity is equal to 1 if the patient's heart rate is equal to or more than 75 beats per minute and otherwise equal to 0, and

wherein a eighth quantity is equal to 1 if the patient's gender is male and otherwise equal to 0.

Preferably, the above set of eight biological parameters comprises a further variable in form of an indicator of myocardial function or myocardial performance, wherein the indicator of myocardial function or myocardial performance is defined by the patient's left ventricular ejection fraction (LVEF) in % of volume per volume, and wherein the biological parameters are set into correlation by a sum of at least nine quantities, the sum thereby providing the simplified risk score $X_{1a\#}$.

The ninth quantity is defined by the patient's left ventricular ejection fraction (LVEF) in % of volume per volume, wherein the ninth quantity is equal to 1 if the left ventricular ejection fraction (LVEF) is moderately reduced, equal to 2 if the left ventricular ejection fraction is severely reduced and otherwise equal to 0, wherein a moderately reduced left ventricular ejection fraction (LVEF) is defined by a value of 30 - 44 in % of volume per volume, and wherein a severely reduced left ventricular ejection fraction (LVEF) is defined by a value of below 30 in % of volume per volume.

It has further been found that a particular mortality risk score can be correlated with a particular risk class of mortality, i.e. with a class of low risk of mortality, a class of intermediate (or moderate) risk of mortality and a class of high risk of mortality.

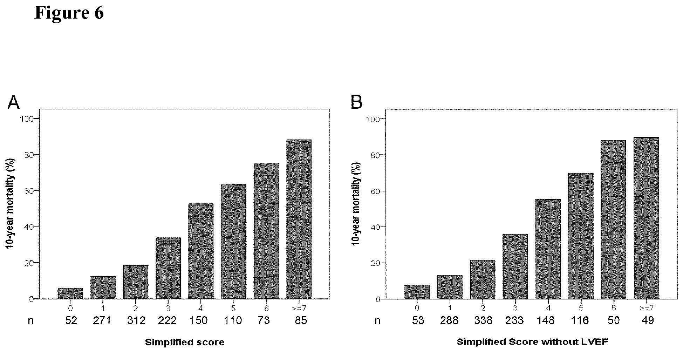

This classification is applicable for all mortality risk scores, in particular for any of the risk scores $X_1$, $X_{1a}$, $X_{1\#}$ and/or $X_{1a\#}$, the classification of which is further exemplified by the Examples of the present invention, in particular by Figures 3 and 6.

Using the simplified risk score $X_1$, $X_{1a}$, $X_{1\#}$ or $X_{1a\#}$, the risk of mortality was calculated by dividing the number of patients who died within a certain time window, in particular within the time window of 10 years, by the number of patients who had initially been classified with this risk score. Thereby, different risk scores could be correlated with different risks of mortality, in particular with different risk score classes as exemplified in the Examples of the present invention.

Therefore, a simplified risk score $X_1$ or $X_{1a}$ of equal to 0 indicates a low risk of mortality, while a simplified risk score $X_1$ or $X_{1a}$ of equal to 1 or equal to 2 indicates an intermediate risk of mortality and a simplified risk score $X_1$ or $X_{1a}$ of equal to or more than 3 indicates a high risk of mortality.

Here, a low risk of mortality means a risk of mortality of below 20 %, an intermediate risk of mortality means a risk of mortality of between 30 to 50 %, preferably of about 40 %, while a high risk of mortality means a risk of mortality of at least 60 %.

According to another preferred aspect, a simplified risk score $X_{1\#}$ or $X_{1a\#}$ of equal to 0 or equal to 1 indicates a low risk of mortality, while a simplified risk score $X_{1\#}$ or $X_{1a\#}$ of equal to 2 or equal to 3 indicates a moderate risk of mortality, and a simplified risk score $X_{1\#}$ or $X_{1a\#}$ of equal to or more than 4 indicates a high risk of mortality.

A low risk of mortality means a risk of mortality of below 20 %, a moderate risk of mortality means a risk of mortality of between 30 to 40 %, preferably of below 40 %, while a high risk of mortality means a risk of mortality of at least 50 %.

In another preferred embodiment of the present invention, the risk score is a weighted risk score $X_2$ and the biological parameter are set into correlation by using the following formula, thereby providing the weighted risk score $X_2$:

$$X_2 = (\text{variable } 1 - 4.14) * 2.33 + (\text{variable } 2) * 0.42 + (\text{variable } 3 - 1.73) * - 1.05 + (\text{variable } 4 - 0.68) * 1.27 + (\text{variable } 5 - 4.21) * 1.47 + (\text{variable } 6 - 1.85) * 1.79,$$

wherein

variable 1 is defined by the log value of the patient's age in years,

variable 2 is defined as equal to 1 if the left ventricular ejection fraction is moderately reduced, equal to 2 if the left ventricular ejection fraction is severely reduced and otherwise equal to 0, wherein a moderately reduced left ventricular ejection fraction is

defined by a value of 30 - 44 in % of volume per volume, and wherein a severely reduced left ventricular ejection fraction is defined by a value of below 30 in % of volume per volume,

variable 3 is defined by the log value of the patient's blood concentration of serum cholinesterase in Units/ml,

variable 4 is defined by the log value of the patient's blood concentration of serum creatinine in mg/dl,

variable 5 is defined by the log value of the patient's heart rate in beats per minute, and

variable 6 is defined by the log value of the patient's blood concentration of glycated haemoglobin HbAlc in % of total haemoglobin.

Here, the "numeric values in the formula providing the weighted risk score $X_2$" are to be understood as the following numeric values: 4.14, 2.33, 0.42, 1.73, -1.05, 0.68, 1.27, 1.47, 1.85 and 1.79.

Equally preferred is that all numeric values in the formula providing the weighted risk score $X_2$ are defined without any deviation.

Moreover, in the context of the present invention, all log values are calculated to the basis e.

As with the simplified risk score $X_1$ and $X_{1a}$, it has surprisingly been found that also the weighted risk score can be correlated with different classes of the risk of mortality, preferably with a class of low risk of mortality, a class of intermediate risk of mortality and a class of high risk of mortality.

Therefore, in a preferred embodiment of the method of the present invention, a weighted risk score $X_2$ in the range of less than or equal to 0 indicates a low risk of mortality, a weighted risk score $X_2$ in the range of 0 to 1 indicates an intermediate risk of mortality, and a weighted risk score $X_2$ in the range of more than or equal to 1 indicates a high risk or mortality, as exemplified by the examples of the present invention.

In the context of the present invention, it is to be understood that the term "equal to" may comprise a deviation of ± 0.5, preferably of ± 0.25, more preferably of ± 0.1.

Moreover, it is to be understood that those patients with a weighted risk score $X_2$ in the range of less than or equal to 0 fall into the 1st tertile of the given cohort or population (minimum to 33rd percentile), those patients with a weighted risk score $X_2$ in the range of 0 to 1 fall into the 2nd tertile of the cohort (33rd percentile to 66th percentile), while those patients with a weighted risk score $X_2$ in the range of more than or equal to 1 fall into the 3rd tertile of the cohort

(66th percentile to maximum).

The term "percentile" generally means the value of a variable below which a certain percent of observations fall. For example, the 33rd percentile is the value below which 33 percent of the observations may be found. In the present invention, the cut off values off the 1st, 2nd and 3rd tertile have been determined as exemplified in the examples of the present invention.

In yet another preferred embodiment, the risk score is a weighted risk score $X_{2\#}$ and the biological parameters are set into correlation by using the following formula, thereby providing the weighted risk score $X_{2\#}$:

$$X_{2\#} = (\text{variable } 1 - 4.151) * 2.69 + (\text{variable } 2 - 1.848) * 1.32 + (\text{variable } 3 - 4.221) * 0.633 + \text{variable } 4 * 0.182 + (\text{variable } 5 - 5.786) * 0.345 + (\text{variable } 6) * - 0.495 + (\text{variable } 7 - 3.168) * 0.172 + (\text{variable } 8 - 2.688) * -0.492 + (\text{variable } 9 - 0.019) * 0.593,$$

wherein

variable 1 is defined by the log value of the patient's age in years,
variable 2 is defined by the log value of the patient's blood concentration of glycated haemoglobin HbA1c in % of total haemoglobin,
variable 3 is defined by the log value of the patient's heart rate in beats per minute,
variable 4 is defined as equal to 1 if the patient's left ventricular ejection fraction (LVEF) is moderately reduced, equal to 2 if the left ventricular ejection fraction (LVEF) is severely reduced and otherwise equal to 0, wherein a moderately reduced left ventricular ejection fraction is defined by a value of 30 - 44 in % of volume per volume, and wherein a severely reduced left ventricular ejection fraction is defined by a value of below 30 in % of volume per volume,
variable 5 is defined by the log value of the patient's blood concentration of N-terminal pro-B-type natriuretic peptide in ng/L,
variable 6 is defined as equal to 1 if the patient's gender is male and equal to 0 if the patient's gender is female,
variable 7 is defined by the log value of the patient's blood concentration of renin in pg/ml,
variable 8 is defined by the log value of the patient's blood concentration of 25(OH) vitamin $D_3$ in ng/L,
variable 9 is defined by the log value of the patient's blood concentration of cystatin C in mg/L.

Here, the "numeric values in the formula providing the weighted risk score $X_{2\#}$ are to be understood as the following numeric values: 4.151, 2.69, 1.848, 1.32, 4.221, 0.633, 0.182, 5.786, 0.345, - 0.495, 3.168, 0.172, 2.688, - 0.492, 0.019, 0.593.

Equally preferred is that all numeric values in the formula providing the weighted risk score $X_{2\#}$ are defined without any deviation.

As with the simplified risk score $X_{1\#}$ and $X_{1a\#}$, it has been found in the context of the present invention that also the weighted risk score $X_{2\#}$ can be correlated with different risk classes of mortality, preferably with a class of low risk of mortality, a class of intermediate (or moderate) risk of mortality, and a class of high risk of mortality.

Accordingly, in a preferred embodiment of the present invention, a weighted risk score $X_{2\#}$ in the range of less than or equal to 0 indicates a low risk of mortality, a weighted risk score $X_{2\#}$ in the range of 0 to 1 indicates an intermediate (or moderate) risk of mortality, and a weighted risk score $X_{2\#}$ in the range of more than or equal to 1 indicates a high risk or mortality.

In the context of the present invention, the cardiovascular disease is a stable coronary artery disease.

The term "coronary artery disease" or "CAD" refers to a condition also known as atherosclerotic heart disease. This condition is characterized by the accumulation of atherosclerotic plaque at the walls of the coronary arteries. Once the accumulation exceeds a certain level, the blood flow through the artery is impaired. This condition causes Angina pectoris and may cause myocardial infarction, especially if the plaque ruptures. Common intervention to treat CAD comprises percutaneous coronary intervention (PCI) and coronary artery bypass graft surgery.

In patients with "stable coronary artery disease", atherosclerosis is characterized by fibrous proliferation in medium- and large-sized arteries mainly driven by lipid accumulation and eventually resulting in calcification of the vessel wall. Here, atherosclerosis leads to a gradual luminal narrowing over decades. Patients with stable coronary artery disease differ significantly in their long term prognosis as compared to patients with acute coronary syndromes (ACS). That is, in patients with stable CAD, the rate of major cardiovascular events is much lower with an estimated annual rate of 1-2 %, with the consequence that the prediction of the short-term and the intermediate-term risk is less relevant. Detailed information about the clinical picture of stable coronary artery disease including its therapy is, for example, described in the ICSI Health Care Guidelines, 14th Edition 2011.

As found in the context of the present invention, the risk scores calculated by the methods of the present invention demonstrated an excellent discriminatory power for 10-years survival with a C-statistic value of 0.77 (P< 0.001), thereby providing for a discriminatory ability to predict 10-year mortality which is significantly better than those of the conventional risk scores known in the art (see, for example, Marschner et al., 2001,. J Am College Cardiol, 38: 56-63).

Accordingly, in a further preferred embodiment, the risk score of the present invention is indicative for mortality of the patient within the next 3 years, preferably within the next 5 years, more preferably within the next 10 years.

In yet another preferred embodiment, the risk score determined by the methods of the present invention is indicative for a treatment of secondary prevention, wherein said treatment is in form of administered drugs.

A treatment of secondary prevention includes the administration of a therapeutically effective dose or pharmaceutically active compound in form of a pharmaceutical composition, e.g. a drug, which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The term "drug" generally means any composition formulated in solid, liquid (or gaseous) form which can be administered to a patient. Said composition generally comprises a therapeutically active compound optionally together with suitable auxiliary compounds such as diluents or carriers or further ingredients. In this context, it is distinguished between auxiliary compounds, i.e. compounds which do not contribute to the effects elicited by the compound upon application of the composition for its desired purpose, and further ingredients, i.e. compounds which contribute a further effect or modulate the effect of the therapeutic effective ingredient. Suitable diluents and/or carriers depend on the purpose for which the composition is to be used and the other ingredients. The person skilled in the art can determine such suitable diluents and/or carriers without further ado. Examples of suitable carriers and/or diluents are disclosed elsewhere herein. Drugs suitable for oral administration may be presented as discrete units such as capsules, cachets, tablets, or lozenges. Other compositions include suspensions in aqueous liquors or non-aqueous liquids such as an syrup, an elixir, or an emulsion.

As used herein, an effective amount of the administered drug is a dosage large enough to produce the desired therapeutic effect to reduce the risk of mortality. An effective amount is not, however, a dosage so large as to cause adverse side effects. Generally, an effective amount may vary with the patient's age, condition, weight and sex, as well as the extent of the condition being treated, and can be determined by one of skill in the art. The dosage may be adjusted by the individual practitioner in the event of any complication.

Preferably, these drugs are for treating hyperlipidemia, for regulating blood pressure, for modifying the heart rate and/or for providing glycaemic control in diabetic patients.

The term "hyperlipidaemia" is also referred to in the art as "hyperlipoproteinemia" and/or "hyperlipidaemia" and generally means a disease which involves abnormally elevated levels of any or all lipids and/or lipoproteins in the blood. Hyperlipidaemias are divided in primary and secondary subtypes. Primary hyperlipidaemia is usually due to genetic causes (such as a mutation in a receptor protein), while secondary hyperlipidaemia arises due to other underlying causes such as diabetes. Lipid and lipoprotein abnormalities are common in the general population, and are regarded as a modifiable risk factor for cardiovascular disease due to their influence on atherosclerosis.

Preferably, treating hyperlipidaemia means a treatment which results in a reduction of the patient's blood lipids and/or lipoproteins, preferably in reducing the concentration of cholesterol.

The term "glycaemic control" generally refers to any medical treatment that aims at controlling the levels of blood glucose (blood sugar) in a person with diabetes mellitus. Blood glucose levels can be measured by means of a glucose meter, with the result either indicated in mg/dL (milligrams per deciliter in the USA) or mmol/L (millimoles per litre in Canada and Europe) of blood. The average glucose level of a healthy person is around 4.5 to 7.0 mmol/L (80 to 125 mg/dL).

Advantageously, according to the claims, the present invention provides reliable methods for determining the risk of mortality in patients with cardiovascular disease. The identification of high risk patients allows for a closer monitoring of this group so that preventive treatments can be administered to those patients with the greatest need. This suggests specific preventive measures of secondary treatment: patients with a determined intermediate (or moderate) or high risk of mortality should receive therapies that aim, for example, at reducing the concentration of cholesterol in the blood, at reducing the patient's blood glucose concentration, or at reducing the patient's blood pressure. Thus, the present invention contributes to the development of individualized treatment regimens.

Therefore, in a preferred embodiment of the invention, the second prevention treatment is in form of administered drugs, wherein the drugs are for reducing the patient's blood concentration of cholesterol, for reducing the patient's blood pressure, and/or for reducing the patient's blood glucose concentration.

The treatment of secondary prevention is preferably applied to patients for whom an intermediate (or moderate) risk of mortality or a high risk of mortality has been determined by the methods of the present invention.

Accordingly, in a preferred embodiment, the risk score determined by the methods of the present invention is indicative for a treatment of secondary prevention of patients with an intermediate (or moderate) risk of mortality, or of patients with a high risk of mortality.

More preferably, the treatment of secondary prevention is applied more intensively in patients with a high risk of mortality than in patients with an intermediate risk of mortality. Consequently, the treatment of secondary prevention may be the more intense, the higher the patient's risk of mortality has been determined by the methods of the present invention.

In another aspect, the present invention provides a computer program product according to the claims directly loadable into the internal memory of a digital computer, comprising software code portions for implementing the calculation of the mortality risk score according to the present invention when said product is run on a computer.

According to the invention, a computer program product according to the claims is provided for performing one of the previously explained methods, when the product is run on a computer. The computer program product is preferred to be directly loadable into the internal memory of a digital computer and comprises software code portions for implementing the calculation of the risk score.

The computer program product may be a computer program preferably stored on a machine readable storage medium like RAM, ROM, or on a removable CD-ROM, flash memory, DVD or USB-stick. The computer program may be provided on a server to be downloaded via for example a data network such as the internet or another transfer system such as a phone line or a wireless transfer connection. Additionally, or alternatively, the computer program product may be a network of computer implemented computer programs such as a client/server system or a cloud computing system, an embedded system with a computer program or an electronic device like a smart phone or a personal computer on which a computer program is stored, loaded, running, exercised, or developed.

In one implementation, the information on the set of biological parameters obtained from the patient is entered into an input device of a computer, an embedded system, an electronic device or a smart phone via a keyboard, a touch screen or a voice interface. In another implementation, the information on the set of biological parameters is measured by adequate measuring devices and transmitted via the internet or another transfer system such as a phone line or a wireless transfer connection to a remote station such as a computer.

In one implementation, the information on the calculated risk score is provided on an output medium, stored in a memory means or transferred to a remote station. In one embodiment, the information on the risk score is displayed on a screen or display as output medium.

Preferably, the computer program product comprises software code portions for implementing the calculation of the weighted risk scores as defined in the context of the present invention.

In yet another preferred aspect, the computer program product of the present invention comprises software code portions for implementing the calculation of the simplified risk scores $X_1$, $X_{1a}$, $X_{1\#}$ and/or $X_{1a\#}$ as defined in the context of the present invention.

It is to be understood that the definitions and explanations of the methods, measurements, and terms made above apply mutatis mutandis for all aspects described in this specification in the following except as otherwise indicated.

The following Figures and Examples are intended to illustrate various embodiments of the present invention. As such, the specific modifications discussed therein are not to be understood as limitations of the scope of the invention.

## FIGURES

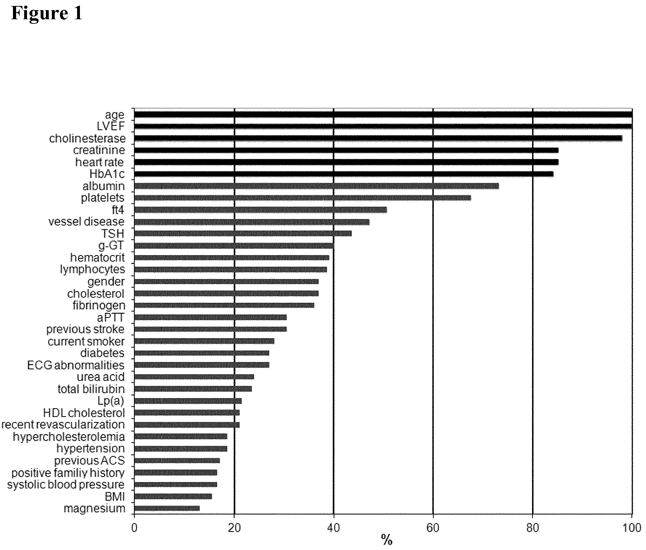

**Figure 1:** Variable selection by a bootstrap resampling procedure based on Cox regression analysis - Variables selected in more than 80 % of all repeats (black bars) were included in the final multivariable model. Hbalc values of the complete study population were used for the bootstrap resampling procedure. Fasting glucose, Non-HDL-cholesterol, LDL-cholesterol, hypertriglyceridemia and triglycerides, iron, protein, ASAT and ALAT, C-reactive protein, thrombin time, mean platelet volume, leukocyte count and relative neutrophil count were excluded before bootstrap selection due to close correlation with other variables entering the bootstrap selection. A post-hoc analysis showed that pharmacological treatment including aspirin, thienopyridines, renin-angiotensin-aldosterone system inhibitors, beta-blockers and statins would not qualify for the final model.

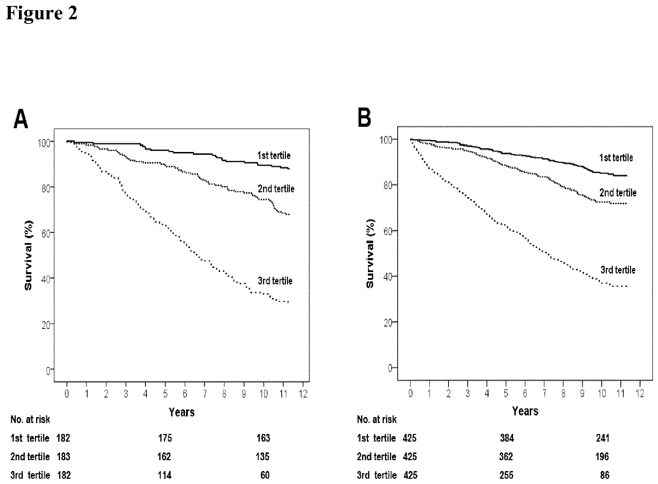

**Figure 2:** Kaplan-Meier estimates of all-cause mortality in stable CAD - Kaplan-Meier estimates of all-cause mortality according to tertiles of the VILCAD risk score for stable CAD for the derivation (A) and validation cohort (B, both P<0.001 between all tertiles, log rank test)

**Figure 3:** Risk Prediction of simplified risk score - The bars show 10-year mortality stratified by the simplified score in the derivation (A, B) and validation (C, D) cohort with (A, C) or without inclusion of cholinesterase (B, D). The following cut-off values were applied for the generation of the simplified score:

[age $\geq$ 75 years (82nd percentile)=2] + [LVEF moderate=1, severe=2] + [Hbalc $\geq$ 6.5% in patients with known diabetes (66th percentile)=1] + [creatinine $\geq$ 1.3 mg/dl (86th percentile)=1] + [heart rate $\geq$ 75 bpm (70th percentile)=1] + [cholinesterase $\leq$ 4.4 kU/L (10th percentile)=1 for A and C]. Percentiles derived from the derivation cohort.

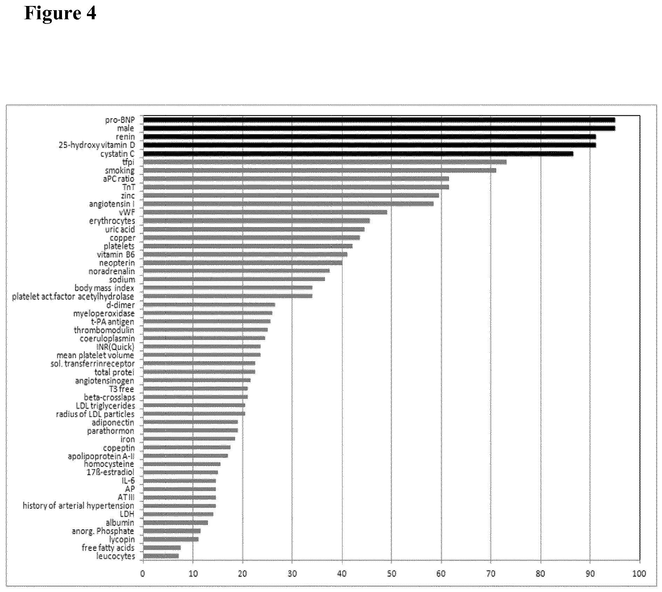

**Figure 4:** Variable selection by a bootstrap resampling procedure based on Cox regression analysis (cut off level for selection: 80%) - To avoid collinearity the following variables were selected for the bootstrap procedure due to their highest univariate predictive value (defined by the hazard ratio for 1-SD change derived from Cox regression) from clusters with close correlations (r > 0.4): Cystatin (NOT selected: BUN, creatinine, ADMA, SDMA, eGFRMDRD), free fatty acids, LDL triglycerides and platelet act. factor acetylhydrolase (NOT selected: total cholesterol, HDL triglycerides, lipoprotein(a), free glycerol, apolipoprotein A-I, HDL cholesterol ester, HDL phospholipid, HDL cholesterol, cholesterol ester, LDL cholesterol, apolipoprotein E, LDL apolipoprotein B, apolipoprotein B, LDL phospholipid, triglycerides, HDL free cholesterol, apolipoprotein C-III, a-Tocopherol, phospholipid, VLDL apolipoprotein B, VLDL cholesterol ester, VLDL cholesterol, LDL free cholesterol, VLDL free cholesterol, apolipoprotein A-II), body mass index (NOT selected: waist-to-hip ratio), antithrombin III (NOT selected: retinol), lycopin (NOT selected: alpha_carotin, beta-carotin, lutein + zeaxanthin, all-trans beta-carotin, cis beta-carotin, beta_cryptoxanthin), noradrenalin (NOT selected: adrenalin), iron (NOT selected: ferritin, transferrin), erythrocytes (NOT selected: hematocrit, hemoglobin, MCV, MCH, MCHC), factor VII (NOT selected: activated factor VII), LH (NOT selected: FSH), IL-6 (NOT selected: hsCRP, fibrinogen, serum amyloid A, LBP), INR(Quick) (NOT selected: endogenous thrombin potential (ETP), prothrombin fragment 1+2, aPTT), 56glycosylated hemoglobin (NOT selected: fasting glucose, glucose 1 and 2h post OGTT, fasting insulin, insulin 1 and 2h post OGTT, proinsulin 1 and 2h post OGTT, C-peptide 1 and 2h post OGTT), cholinesterase (NOT selected: gamma GT, ALT, AST, total bilirubin), beta-crosslaps (NOT selected: osteocalcin), t-PA antigen (NOT selected: t-PA activity, PAI-1 activity, PAI-1 antigen), NT pro-brain natriuretic peptide (NOT selected: homoarginine), renin (NOT selected: aldosterone), free T3 (NOT selected: free T4, TSH), vitamin B6 (NOT selected: folic acid, vitamin B12, vitamin B1), 25-hydroxy vitamin D (NOT selected: 1-25-dihydroxy vitamin); quick prothrombin time; BUN, blood urea nitrogen; HDL, high-density lipoprotein; BMI, body mass index; LDL, low-density lipoprotein; ALT alanin-aminotransferase; Variables with a high univariate hazard ratio (NT pro-BNP, TnT, cystatin C, copeptin, neopterin) were included in the bootstrapping resampling procedure despite correlation coefficients > 0.4. In case of close correlations selection of the respective variables were tested with and without the correlating variable.

**Figure 5:** Kaplan-Meier estimates of mortality - Kaplan-Meier estimates for tertiles of biomarker score with (A,C) and without LVEF (B,D) for all-cause mortality (A,B) and cardiovascular mortality (C,D) (all P < 0.001 between all tertiles, log rank test) .

**Figure 6:** Risk prediction of simplified risk score - The bars show 10-year mortality stratified by the simplified biomarker score with (A) and without LVEF (B). The following cut-off values were applied for the generation of the simplified score:

[male = 1] + [age $\geq$ 75 years (82nd percentile) = 2] + [heart rate $\geq$ 75 bpm (70th percentile) = 1] + [LVEF moderate =1, severe =2] + [NT-pro-BNP > 400 ng/L (58th percentile) =1, >2000 ng/L (89th percentile)=2] + [Hbalc $\geq$ 6.5% in patients with known diabetes (66th percentile) =1] + [renin >50 pg/mL (73rd percentile)=1) + [25-OH vitamin $D_3$ <10 ng/L (26th percentile) =1] + [cystatin C > 1.2 mg/L (84th percentile)=1]. Percentiles derived from the derivation cohort.

## EXAMPLES

## Example 1

Aims: Previous risk assessment scores for patients with coronary artery disease (CAD) have focused on primary prevention and patients with acute coronary syndrome. However, especially in stable CAD patients improved long-term risk prediction is crucial to efficiently apply measures of secondary prevention. We aimed to create a clinically applicable mortality prediction score for stable CAD patients based on routinely determined laboratory biomarkers and clinical determinants of secondary prevention.

Methods and Results: We prospectively included 547 patients with stable CAD and a median follow-up of 11.3 years. Independent risk factors were selected using bootstrapping based on Cox regression analysis. Age, left ventricular function, serum cholinesterase, creatinine, heart rate, and HbAlc were selected as significant mortality predictors for the final multivariable model. The Vienna and Ludwigshafen Coronary Artery Disease (VILCAD) risk score based on the aforementioned variables demonstrated an excellent discriminatory power for 10-year survival with a C-statistic of 0.77 (P<0.001), which was significantly better than an established risk score based on conventional cardiovascular risk factors (C-statistic = 0.61, P<0.001). Net reclassification confirmed a significant improvement of individual risk prediction by 34.8 % (95 % confidence interval: 21.7-48.0 %) compared to the conventional risk score (P<0.001). External validation of the risk score in 1275 participants of the Ludwigshafen Risk and Cardiovascular Health (LURIC) study (median follow-up 9.8 years) achieved similar results (C-statistic = 0.73, P<0.001).

Conclusions: The VILCAD score based on a routinely available set of risk factors, measures of cardiac function and comorbidities outperforms established risk prediction algorithms and might improve the identification of high-risk patients for a more intensive treatment.

Atherosclerosis is a chronic immune inflammatory disease with stable and unstable clinical presentation (1). In patients with stable coronary artery disease (CAD), atherosclerosis is characterized by fibrous proliferation in medium- and large-sized arteries mainly driven by lipid accumulation and eventually resulting in calcification of the vessel wall. Consequently, atherosclerosis leads to a gradual luminal narrowing over decades. In contrast, acute coronary syndromes (ACS) are driven by a dynamic component, mostly atherosclerotic plaque rupture followed by thrombosis and coronary occlusion (2). Although, stable CAD and ACS represent two entities of the same disease, they differ distinctly in their long-term prognosis (3). In ACS patients major cardiovascular events occur frequently during the following months. Therefore, various risk assessment scores including the Platelet glycoprotein IIb/IIIa in Unstable Angina: Receptor Suppression Using Integrilin (PURSUIT) Risk score, the Thrombolysis in Myocardial Infarction (TIMI) Risk score or the Global Registry of Acute Coronary Events (GRACE) Risk score have been developed (3). In stable CAD patients the rate of major cardiovascular events is much lower with an estimated annual rate of 1 to 2% (3, 4), and thus prediction of the short-term and the intermediate-term risk is less relevant. In this patient population it is more important to have sufficient diagnostic tools for the estimation of the accumulating long-term risk to guarantee an efficient application of measures of secondary prevention including the lifelong administration of drugs, associated with reduced readmission rates and overall mortality (5, 6). However, there is currently no established mortality prediction score for patients with stable CAD. So far, only a score published by Marschner et al. which has initially been developed for patients with ACS has been proposed for risk prediction in stable CAD patients (3). This risk score encompasses cardiovascular risk factors such as age, gender, smoking habits, hypertension, diabetes and history of previous cardiovascular events, but does not include laboratory markers except for lipoprotein levels (7). While these established cardiovascular risk factors are crucial for risk estimation of the development of CAD, different clinical or laboratory variables may become important for the long-term outcome in patients with manifest CAD. Notably, renal and liver function may be relevant, particularly if these patients receive a set of drugs over decades (8, 9).

The aim of our study was to assess the independent predictive power of known clinical risk factors and routinely tested laboratory biomarkers for long-term mortality in stable CAD patients. We further aimed to create a clinically applicable mortality prediction score for this specific patient population based on a stringent statistical selection procedure.

**Methods**

Derivation cohort

We prospectively included 1152 consecutive CAD patients referred to the Department of Cardiology of the Vienna General Hospital between November 1999 and August 2000 (10). Of these, 547 patients with stable CAD were selected for this study. The study protocol complies with the Declaration of Helsinki and was approved by the Ethics Committee of the Medical University of Vienna.

Validation cohort

We used the LUdwigshafen Risk and Cardiovascular Health (LURIC) study for external validation of risk prediction. The detailed study protocol has been previously described (11). In brief, 3500 patients referred to the Heart Center of Ludwigshafen for coronary angiography were enrolled between July 1997 and January 2000. For the purpose of this study we selected 1275 patients with proven CAD who presented in a stable condition.

Clinical definitions

Stable CAD was defined as angiographical evidence of stenosis of an epicardial coronary artery of ≥60% in the derivation cohort and of ≥50% in the validation cohort. Cardiovascular risk factors such as hypertension, diabetes mellitus, current smoking, and lipid disorders were recorded according to the respective guidelines (10). Left ventricular ejection fraction (LVEF) was estimated using the Simpson method and categorized into normal (≥55%), mildly reduced (45-54%), moderately reduced (30-44%) and severely reduced (<30%). Blood samples were analyzed without freezing according to local laboratory standard procedure.

Study endpoints and Follow-up

The primary study endpoint was all-cause mortality and obtained by screening the national register of death, including screening for the cause of death (according to the International Statistical Classification of Diseases and Related Health Problems 10th Revision). In the derivation cohort post-mortem examination was performed in 34% of patients to verify the cause of death. In the validation cohort (LURIC) information was obtained from local registries and death certificates were used to classify who died of cardiovascular and non-cardiovascular cause as previously described (12).

Statistical analysis

Continuous data were presented as median and interquartile range, discrete data as counts and percentages. The Mann-Whitney-U test and chi-square test were used for comparisons between groups, as appropriate. Cox proportional hazard regression analysis was applied to assess the effect of variables on survival. Continuous variables underwent log transformation before entering analysis. A bootstrap resampling procedure was used to identify best-fitting variables for the multivariable Cox regression model. Before inclusion of variables into the bootstrapping procedure collinearity between biomarkers was assessed using Pearson correlation coefficients (data not shown). In case of close correlation only the strongest univariate predictor of a cluster (defined by the highest univariate hazard ratio for an increase of 1 standard deviation (SD)) entered the selection procedure. Samples with a size of 80% of the original cohort were chosen for repeats. 100 repeats with forward selection and 100 repeats with backward selection (with a P<0.1 for selection) were performed. Variables selected in more than 80% of all repeats were included in the final multivariable model. The discriminatory power of the designed risk scores was assessed using Harrell's C-statistic. For further analysis we stratified the cohort by tertiles of the multimarker risk scores. An improvement of individual risk prediction was examined by the net reclassification improvement (NRI) as described by Pencina et al. (13). Kaplan-Meier analysis (log-rank test) was applied to verify the time-dependent discriminative power of risk scores. Cut-off values for a simplified score using categorized variables were estimated using chi-square values derived from Martingale residuals of the Cox model for all possible cut-off values. For optimized clinical use these calculated cut-off values were rounded and extreme values were avoided. Two-sided P-values <0.05 were used to indicate statistical significance. SPSS 18.0 (IBM SPSS, USA) and STATA 11 (StataCorp LP, USA) were used for all analyses.

## Results

Baseline characteristics and univariate survival analysis: We prospectively enrolled 547 stable CAD patients into our derivation cohort (Vienna). Thirty-nine percent of all patients (n=211) died during a median follow-up of 11.3 years (IQR: 6.9- 11.5) corresponding to 4974 overall person-years of follow-up. Fifty-seven percent of deaths in patients were due to cardiovascular causes. The validation cohort (LURIC) includes 1275 prospectively enrolled stable CAD patients with a median follow-up of 9.8 years (IQR 7.0- 10.5). Of these patients 35% (n=499) died during the observation period (primary endpoint), 51% died from cardiovascular causes. Baseline characteristics for both study cohorts are displayed in Supplemental Table 1. In the univariate analysis, the strongest adverse risk factors for long-term outcome in stable CAD patients were age with a hazard ratio (HR) of 1.94 per 1-SD increase in the derivation cohort and a HR of 1.84 in the validation cohort and LVEF with a HR of 1.67 for a reduction of one semiquantitative category in the derivation cohort and a HR of 1.75 in the validation cohort, respectively. Cholinesterase had a strong inverse effect on mortality with a HR of 0.57 per 1-SD increase in the derivation cohort and of 0.72 in the validation cohort (Table 1).

Bootstrapping results and multivariable survival analysis: After variable selection by a bootstrap technique age, LVEF, serum cholinesterase, creatinine, heart rate and HbAlc were selected as significant mortality predictors for the final multivariable model (Figure 1). In this multivariable model age and LVEF remained the strongest adverse risk factors and cholinesterase the strongest inverse risk factor (Table 1).

Risk score design and internal validation: In a second step, we computed a weighted risk score, the Vienna and Ludwigshafen Coronary Artery Disease (VILCAD) risk score, for 10-year survival using the aforementioned variables obtained by the bootstrapping procedure (see Supplemental Method 1 for the formula). The C-statistic demonstrated an excellent discriminatory power of the VILCAD risk score for 10-year survival with a value of 0.77 (P<0.001). The discriminatory ability of our score to predict 10-year mortality was significantly better than that of a conventional risk score published by Marschner et al. (C-statistic 0.61; P<0.001 for comparison of the two scores) (7). The predictive value of the new multimarker score was also superior to that of the GRACE score (C-statistic=0.67; P<0.001 for the comparison between the two scores) (14). Kaplan Meier analysis confirmed a high discriminative power when plotting tertiles of the VILCAD risk score (P<0.001 between all tertiles, Figure 2A) with 10-year survival rates of 90%, 75%, and 33% in the first, second and third tertile of the score. The NRI showed an improvement in individual risk classification of 34.8% (95% confidence interval [CI]: 21.7-48.0%) compared to the conventional risk score published by Marschner et al. (7) (Table 2, P<0.001). With regard to cardiovascular mortality, a comparable trend for the 10-year survival was observed, with survival rates of 94%, 86%, and 52% in the first, second and third tertile of the new risk score (P<0.001 between all tertiles).

External validation: We validated our multimarker risk score in the LURIC study population (11). A C-statistic of 0.73 confirmed the strong and superior discriminatory power of our risk score to predict long-term mortality (P<0.001 for comparisons with the C-statistics of 0.64 for the Marschner risk score (7) and of 0.61 for the GRACE risk score (14)). Kaplan Meier analysis confirmed a high discriminative value when plotting tertiles of the new risk score (P<0.001 between all tertiles, Figure 2B) with 10-year survival rates of 85%, 73%, and 37% in the first, second and third tertile of the score. With regard to cardiovascular mortality, a comparable trend for the 10-year survival was observed, with survival rates of 93%, 83%, and 52% in the first, second and third tertile of the VILCAD risk score (P<0.001 between all tertiles). A NRI of 14.8% (95%CI 6.5-23.2%) confirmed a significant improvement of individual risk classification in the validation cohort compared to the conventional risk score by Marschner et al. (P<0.001, Table 2).

Simplified score

Finally, we designed a simplified risk score for optimal clinical use. This score was based on the same variables as the VILCAD risk score. Continuous variables were dichotomized according to optimal cut-off levels, as described in Figure 3. Two points were assigned to the variables age and LVEF with the strongest predictive value. This simplified multimarker risk score still had a strong predictive value for 10-year mortality with a C-statistic of 0.74 in the derivation cohort and of 0.70 in the validation cohort (both P<0.001 for comparison with the conventional risk score). The simplified score showed a highly discriminative value with 10-year mortality of 11% in patients with 0 points and of 89% in patients with at least 5 points in the derivation cohort and a similar spread in the validation cohort (Figure 3A&3C). As cholinesterase may not be available in all centres we also calculated a simplified risk score without cholinesterase, which showed a similar predictive value in the derivation and validation cohort and a comparable discrimination of mortality risk (Figure 3B&D).

Discussion: We developed a multimarker score for patients with stable CAD providing an excellent discriminatory power for the prediction of 10-year survival. The final set of six variables was selected by a stringent bootstrap resampling procedure from 48 clinical variables and routinely available biomarkers which reflect a broad range of relevant pathophysiological pathways, determinants of heart disease and comorbidities. The clinical relevance of the VILCAD score was further emphasized by a significant improvement of net reclassification by 34.8%, which reflects the proportion of individuals, who may benefit from an improved risk prediction compared to a conventional risk score. Most importantly, we additionally performed an external validation of our risk score in the LURIC study population (11), a large prospective European cohort study, and obtained comparable results confirming the accuracy of our risk score. Despite the large number of published risk scores, model transportability has rarely been evaluated in external study populations but is crucial for the objective and critical evaluation of evolving risk scores (15).

Previous risk assessment scores for CAD patients have pre-dominantly focused on the high-risk patients presenting with ACS ranging from the PURSUIT Risk score over the TIMI Risk score to the GRACE Risk score (3). In comparison, the area under the Receiver Operating Curve (AUC) of the TIMI, PURSUIT and GRACE risk scores ranged from 0.69 to 0.79 for mortality prediction in ACS populations. In contrast to the present study, these risk scores were based on relatively short follow-up times of one year and used the less stringent receiver operating characteristic curves instead of the recommended C-statistic (16). In our study population the GRACE score only reached a C-statistic of 0.67 in the derivation cohort and of 0.61 in the validation cohort and was distinctly weaker than the presented VILCAD score. There is increasing evidence that stable CAD patients differ significantly from patients with ACS in terms of plaque morphology and extent of affection of coronary arteries (17-19). Therefore, it is likely that different risk factors might be relevant with regard to long-term mortality prediction. Thus, ACS risk scores may not be applicable to patients with stable CAD. However, information on long-term prediction of patients with established stable CAD is scarce (20) and, to the best of our knowledge, there is no established mortality prediction score for stable CAD patients (3). One previous risk score published by Marschner et al. included CAD patients with an acute cardiac event 3 to 36 months before inclusion and has recently been proposed as risk assessment score for stable CAD patients (3, 7). The application of the Marschner score in our cohort resulted in a C-statistic of 0.61 in the derivation cohort and of 0.64 in the validation cohort, which was strikingly lower than the C-statistic achieved with the new VILCAD score (P<0.001 for both cohorts). Recently, another multimarker score for stable CAD integrating the novel biomarkers N-terminal pro-B-type natriuretic peptide, growth differentiation factor 15, mid-regional-pro-atrial natriuretic peptide, cystatin C, and mid-regional-pro-adrenomedullin has been published (21). While integration of a comprehensive set of potentially relevant pathomechanisms is supposed to improve risk prediction, the aforementioned score yielded an AUC of only 0.69 with respect to a median follow-up of 3.6 years. Additional costs for the determination of novel biomarkers may limit the use of the aforementioned score in clinical practice.

The relative importance of established cardiovascular risk factors differs in patients with manifest CAD compared to primary prevention strategies (5). In addition to established cardiovascular risk factors, determinants of heart disease such as left ventricular function, heart rate, normal ECG, multi-vessel disease and coronary revascularization status gain importance in secondary risk prediction (3, 22, 23). Moreover, liver and renal function may affect the treatment with established cardiac medication and the outcome of stable CAD patients (8, 9). Accordingly, a wide range of easily available clinical and laboratory markers may be important and were included in the bootstrapping procedure. The final model selected by a stringent statistical method encompassed age, cardiac parameters (LVEF and heart rate), renal function (creatinine), liver function (CHE), and HbA1c, a metabolic measurement of poor glycemic control in diabetic patients. While we confirmed the already known importance of age, LVEF, heart rate, creatinine and HbAlc in cardiovascular disease the role of cholinesterase is less well established. Serum cholinesterase is a non-specific cholinesterase enzyme that hydrolyses different choline esters and has previously been implicated in the development of CAD (24). A previous study by Calderon-Margalit et al. demonstrated that cholinesterase might be a nonspecific risk factor for mortality (24). Moreover, an analysis from our group demonstrated a strong association between decreased cholinesterase and long-term outcome in patients with known CAD, which was stronger in stable CAD patients than in those with ACS (25).

Despite their repeatedly proven accuracy (26), risk scores are underused in clinical practice mainly due to time constraints. Therefore, we also developed a simplified multimarker score optimized for clinical use based on the variables selected by the bootstrapping procedure. The simplified multimarker score divides markers only in two to three categories based on optimized cut-off values as recently propagated by Kooter et al. (27). In contrast to more complex risk scores this simplified multimarker risk score can conveniently be computed by hand without the use of a calculator. This simplified risk score is cost-effective as it comprises routinely determined laboratory measurements and conventional clinical data. The simplified score still had a competitive predictive performance resulting in a C-statistic of 0.74 in the derivation cohort and of 0.70 in the validation cohort which is illustrated by the following comparison: A 74-year-old patient with stable CAD, a creatinine of 1.5 mg/dl but none of the other included risk predictors would reach 1 point in our score and would have a 37% risk of death during the following 10 years (Figure 3). On the contrary, a 77-year old patient with a severely reduced LVEF, and an HbAlc of 6.8% (5 points) would expect an 89% risk of death within the next ten years. The use of easily applicable scores is important as a previous report has shown that multimarker scores improve the accuracy of risk prediction compared to the sole clinical judgment of the treating physician (16). Awareness of the risk of a patient may have important consequences on the choice of treatment options. Aggressive treatment options might be underused as high-risk patients might not be recognized due to an inaccurate risk assessment (16).

Limitations: We are aware of the following limitations of our study: the patient cohort was recruited at a university-affiliated tertiary care center with a high-volume cardiac catheterization unit and may therefore not be representative for stable CAD patients in general. Furthermore, patients were included into the study between 1999 and 2000. Since then secondary prevention strategies have been modified (e.g. drug eluting stents, automated implantable cardioverter defibrillator implantation guidelines, cardiac resynchronization therapy, and heart rate modification). These new prevention strategies might affect the accuracy of our risk score. Moreover, different laboratory markers may be available in different health care systems. Yet, the performance of the score was robust when variables such as cholinesterase were excluded which may not be routinely available in other centers. Third generation troponin T assays were not available at the time of study enrollment. However, the variable ECG abnormalities suggesting (clinically silent) ischemia were not selected for the final model. Also, brain natriuretic peptide was not routinely measured in stable CAD patients. However, myocardial function measured by echocardiography was selected most frequently and was one of the strongest risk factors in the final model. While these evolving biomarkers may improve the prediction of long-term mortality, a C-statistic of greater 0.77 has been rarely achieved for long-term mortality. Therefore, it is improbable that the predictive value of the new VILCAD score will be significantly improved by a single biomarker. Finally, the analysis of net reclassification is limited by the lack of generally accepted risk categories for long-term mortality in this patient cohort.

Conclusion: A distinct set of clinical and laboratory variables seem to be relevant to determine the long-term survival of patients presenting with stable CAD. The presented

VILCAD score fills a gap in risk prediction of these patients. The selected variables encompass age, cardiac, renal and liver function, and HbA1c, a measurement of poorly controlled diabetes. The presented risk score may improve the precision of the prediction of survival as evidenced by its high discriminatory power and its confirmation in an external validation cohort. One may speculate that improved risk prediction may help to more specifically tailor possible treatment options in secondary prevention of CAD and to specifically select high-risk patients who may benefit from an intensified treatment.

**Table 1: Unadjusted and adjusted effects on mortality** - Cox proportional hazard model of variables selected by bootstrapping.

Hazard ratios (HR) refer to a 1 - SD increase in continuous variables and to a reduction of one category of LVEF. HRs are adjusted (adj.) for all variables selected by bootstrapping i.e. for age, LVEF, serum cholinesterase, creatinine, heart rate, and HbA1c. SD: standard deviation.

| Vienna study population (n=547) | | | | | |
|---|---|---|---|---|---|
| | SD | Crude HR (95%CI) | P-value | Adj. HR (95%CI) | P-value |
| Age | 11.74 | 1.94 (1.62-2.32) | **<0.001** | 1.64 (1.37-1.95) | **<0.001** |
| LVEF | ---- | 1.67 (1.48-1.88) | **<0.001** | 1.51 (1.34-1.72) | **<0.001** |
| Cholinesterase | 1.43 | 0.57 (0.50-0.66) | **<0.001** | 0.77 (0.68-0.87) | **<0.001** |
| Creatinine | 0.31 | 1.44 (1.30-1.59) | **<0.001** | 1.31 (1.18-1.46) | **<0.001** |
| Heart rate | 13.62 | 1.56 (1.36-1.79) | **<0.001** | 1.30 (1.12-1.50) | **<0.001** |
| HbA1c | 1.13 | 1.36 (1.22-1.51) | **<0.001** | 1.32 (1.17-1.49) | **<0.001** |

| LURIC study population (n=1275) | | | | |
|---|---|---|---|---|
| SD | Crude HR (95%CI) | P-value | Adj. HR (95%CI) | P-value |
| 9.30 | 1.84 (1.64-2.07) | **<0.001** | 1.74 (1.53-1.97) | **<0.001** |
| ---- | 1.75 (1.60-1.91) | **<0.001** | 1.57 (1.43-1.73) | **<0.001** |
| 1.30 | 0.72 (0.67-0.79) | **<0.001** | 0.85 (0.78-0.93) | **<0.001** |
| 0.49 | 1.28 (1.20-1.37) | **<0.001** | 1.20 (1.10-1.30) | **<0.001** |
| 12.45 | 1.31 (1.19-1.43) | **<0.001** | 1.19 (1.08-1.31) | **<0.001** |
| 1.30 | 1.43 (1.32-1.55) | **<0.001** | 1.30 (1.19-1.42) | **<0.001** |

**Table 2: Reclassification table** - Reclassification table comparing the weighted VILCAD risk score with the conventional risk score by Marschner et al., 2001.

Derivation Cohort (Vienna)

| | Conventional risk score | VILCAD risk score | | | |
|---|---|---|---|---|---|
| | Risk class | Low | Moderate | High | Total |
| No event (n=337) | Low | 62% (76) | 29% (36) | 9% (11) | 100% (123) |
| | Moderate | 41% (48) | 41% (47) | 18% (21) | 100% (116) |
| | High | 38% (37) | 42% (41) | 20% (20) | 100% (98) |
| Event (n=210) | Low | 11% (4) | 37% (13) | 51% (18) | 100% (35) |
| | Moderate | 13% (10) | 25% (20) | 62% (49) | 100% (79) |
| | High | 8% (8) | 26% (25) | 66% (63) | 100% (96) |

Validation Cohort (LURIC)

| | Conventional risk score | VILCAD risk score | | | |
|---|---|---|---|---|---|
| | Risk class | Low | Moderate | High | Total |
| No event (n=826) | Low | 58% (227) | 32% (124) | 10% (40) | 100% (391) |
| | Moderate | 34% (85) | 47% (118) | 19% (49) | 100% (252) |
| | High | 28% (51) | 38% (69) | 34% (63) | 100% (183) |

(continued)

| | Conventional risk score | VILCAD risk score | | | |
|---|---|---|---|---|---|
| | Risk class | Low | Moderate | High | Total |
| Event (n=449) | Low | 25% (28) | 33% (37) | 43% (48) | 100% (113) |
| | Moderate | 19% (26) | 29% (39) | 53% (72) | 100% (137) |
| | High | 07% (13) | 24% (47) | 70% (139) | 100% (199) |

**Supplemental Table 1**: Baseline characteristics of patients with stable coronary artery disease of our study population (n= 547) and the LURIC study population (n=1275). Continuous variables are given as median (interquartile range [IQR]). Left ventricular ejection fraction (LVEF); ECG abnormalities are defined as ST-segment deviation and/or T wave inversion; bpm beats per minute.

| | Study population (n= 547) | LURIC study population (n=1275) | p-value |
|---|---|---|---|
| Age, median years (IQR) | 64 (56-73) | 65 (58- 71) | 0.733 |
| Male gender, n (%) | 404 (74%) | 962 (75%) | 0.472 |
| BMI, kg/m$^2$ (IQR) | 27.0 (24.7-29.4) | 27.2 (24.7- 29.8) | 0.411 |
| Multi-vesseldisease, n (%) | 402 (74%) | 745 (58%) | 0.185 |
| Systolic blood pressure, mmHg (IQR) | 135 (120-150) | 145 (128- 161) | **< 0.001** |
| Heart rate, bpm (IQR) | 68 (60-77) | 68 (60- 77) | 0.282 |
| LVEF | | | |
| ≥55%, n (%) | 371 (68%) | 778 (61%) | **0.006** |
| 45-54%, n (%) | 64 (12%) | 207 (16%) | **0.013** |
| 30-44%, n (%) | 51 (09%) | 177 (14%) | **0.007** |
| <30%, n (%) | 58 (11%) | 68 (5%) | **< 0.001** |
| ECG abnormalities, n (%) | 250 (46%) | 351 (28%) | **< 0.001** |
| Medication | | | |
| Platelet inhibitors, n (%) | 465 (85%) | 1252 (98%) | **< 0.001** |
| Acetylsalicylic acid, n (%) | 440 (80%) | ---------- | -------- |
| Thienopyridines, n (%) | 226 (41%) | ---------- | -------- |
| Vitamin K antagonists n (%) | 49 (09%) | 101 (07%) | 0.441 |
| Beta blocker, n (%) | 343 (63%) | 778 (61%) | 0.415 |
| ACE inhibitor/Angiotensin II receptor blockers, n (%) | 354 (65%) | 727 (57%) | **0.004** |
| Digitalis, n (%) | 70 (13%) | 217 (17%) | 0.067 |
| Nitrites, n (%) | 247 (45%) | 359 (28%) | **< 0.001** |
| Statins, n (%) | 363 (66%) | 624 (49%) | **< 0.001** |
| Hypertension, n (%) | 379 (69%) | 807 (63%) | **0.014** |
| Current smokers, n (%) | 221 (40%) | 192 (15%) | **< 0.001** |
| Family history CAD, n (%) | 206 (38%) | 456 (36%) | 0.441 |
| Previous stroke, n (%) | 24 (04%) | 132 (10%) | **< 0.001** |
| Diabetes, n (%) | 155 (28%) | 451 (35%) | **0.003** |

(continued)

| | Study population (n= 547) | LURIC study population (n=1275) | p-value |
|---|---|---|---|
| Hbalc, mg/dl (IQR) | 6.1 (5.7-6.7) | 6.1 (5.7- 6.8) | 0.620 |
| Glucose, mg/dl (IQR) | 107 (95-134) | 94 (84- 111) | **< 0.001** |
| Hypercholesterolemia, n (%) | 372 (68%) | 848 (67%) | 0.533 |
| Total cholesterol, mg/dl (IQR) | 200 (168-228) | 207 (180- 237) | **< 0.001** |
| LDL cholesterol, mg/dl (IQR) | 121 (95-150) | 114 (93- 138) | **< 0.001** |
| HDL cholesterol, mg/dl (IQR) | 45 (38-56) | 37 (32- 45) | **< 0.001** |
| Lp (a), mg/dl (IQR) | 14 (<12-34) | 16.2 (7-41) | **< 0.001** |
| Hypertriglyceridemia, n (%) | 225 (41%) | 596 (47%) | **0.027** |
| Triglycerides, mg/dl (IQR) | 131 (94-194) | 144 (108- 198) | **0.001** |
| Magnesium, mmol/L (IQR) | 0.79 (0.75-0.85) | 0.85 (0.79- 0.91) | **< 0.001** |
| Iron, $\mu$g/dl (IQR) | 88 (69-115) | 91 (70- 115) | 0.233 |
| Creatinine, mg/dl (IQR) | 1.04 (0.94-1.18) | 0.9 (0.8- 1.1) | **< 0.001** |
| Uric acid, mg/dl (IQR) | 6.4 (5.4-7.4) | 4.9 (4.1- 6.0) | **< 0.001** |
| Total bilirubin, mg/dl (IQR) | 0.64 (0.47-0.86) | 0.6 (0.4- 0.8) | **< 0.001** |
| Protein, g/l (IQR) | 74.4 (70.5-77.7) | 6.9 (6.6- 7.3) | **< 0.001** |
| Albumin, g/l (IQR) | 42.8 (40.0-46.2) | 44.0 (41.0- 48.0) | **< 0.001** |
| Cholinesterase, kU/L (IQR) | 5.81 (4.98-6.76) | 5.81 (4.94- 6.68) | 0.692 |
| ASAT, U/L (IQR) | 10 (8-13) | 10 (8-12) | 0.06 |
| ALAT, U/L (IQR) | 13 (9-18) | 12 (9- 17) | **0.04** |
| Gamma-GT, U/L (IQR) | 19 (11-32) | 17 (11-29) | 0.168 |
| C-reactive protein, mg/dl (IQR) | < 0.5 (<0.5- <0.5) | 0.36 (0.09- 0.79) | **< 0.001** |
| APTT, sec (IQR) | 34.2 (31.6-37.9) | 33.0 (31.0- 36.0) | **< 0.001** |
| Thrombin time, sec (IQR) | 14.3 (13.7-15) | ----------------- | |
| Fibrinogen, mg/dl (IQR) | 367 (314-424) | 371 (319- 436) | 0.122 |
| Hematocrit, % (IQR) | 40.6 (38.0-42.7) | 40.9 (38.2- 43.6) | **0.016** |
| Platelets, 1000/$\mu$l (IQR) | 220 (183-261) | 218 (182- 262) | 0.859 |
| Mean platelet volume, fl (IQR) | 10.7 (10.0-11.3) | 9.0 (8.3- 9.6) | **< 0.001** |
| Leukocytes, 1000/$\mu$l (IQR) | 6.8 (5.7-8.1) | 6.72 (5.65- 8.1) | 0.954 |
| Lymphocytes rel. % (IQR) | 25.6 (19.3-31.7) | 26.4 (21.3- 32.0) | 0.054 |
| Neutrophils rel. % (IQR) | 64.6 (58.7-72.0) | 60.5 (54.3- 66.3) | **< 0.001** |
| TSH, $\mu$IU/ml (IQR) | 1.27 (0.83-1.81) | 1.21 (0.73-1.81) | 0.109 |
| fT4, ng/dl (IQR) | 1.35 (1.22-1.49) | 1.35 (1.23- 1.49) | 0.665 |

**Supplemental Method 1:** Generation of the Weighted Multimarker Risk Score

**General:**

Weighted risk score = [V1 – mean of V1] * beta coefficient of V1 + [V2 – mean of V2] * beta coefficient of V2 + [V3 – mean of V3] * beta coefficient of V3 + ……..+ [Vn – mean of Vn] * beta coefficient of Vn.

**Weighted Risk Score:**

= (age - 4.14) * 2.33 + LVEF * 0.42 + (cholinesterase - 1.73) * - 1.05 + (creatinine - 0.68) * 1.27 + (heart rate - 4.21) * 1.47 + (HbA1c - 1.85) * 1.79

Beta coefficients were derived from a multivariable Cox regression model including all variables of the respective model. Continuous variables were log-transformed before inclusion in the score; n: number of variables of the respective risk score; V: variable.

**Supplemental Method 2**: Statistics of Vienna and LURIC cohort

Vienna cohort

| statistics | | |
|---|---|---|
| weighted_score_cad | | |
| N | | 547 |
| minimum | | -3,90 |
| maximum | | 5,97 |
| percentile | 33 | -0,0072 |
| | 66 | 1,0851 |

LURIC cohort

| statistics | | |
|---|---|---|
| score_vienna_wo_alb_plat_master | | |
| N | | 1225 |
| minimum | | -3,14 |
| maximum | | 3,98 |
| percentile | 33 | -0,3159 |
| | 66 | 0,4323 |

**Example 2**

Background: Algorithms to predict the future long-term risk of patients with stable coronary artery disease (CAD) are rare. The VIenna and Ludwigshafen CAD (VILCAD) risk score was one of the first scores specifically tailored for this clinically important patient population. We aimed to refine risk prediction in stable CAD creating a new prediction model encompassing various pathophysiologic pathways. We therefore assessed 135 novel biomarkers for long-term mortality in stable CAD patients.

Methods: We included 1275 stable CAD patients of the LUdwigshafen RIsk and Cardiovascular health (LURIC) study with a median follow-up of 9.8 years to investigate whether the predictive power of the VILCAD score could be improved by the addition of novel biomarkers. Additional biomarkers were selected in a bootstrapping procedure based on Cox regression to determine the most informative predictors for mortality.

Results: The final multivariable model encompassed nine clinical and biochemical markers: age, sex, left ventricular ejection fraction (LVEF), heart rate, NT-proBNP, cystatin C, renin, 25OH-vitamin D3 and HbA1c. The extended VILCAD biomarker score achieved a significantly improved C-statistic (0.78 vs. 0.73; P=0.035) and net reclassification index (14.9%; P<0.001) compared to the original VILCAD score. Omitting LVEF, which might not be readily available, slightly reduced the accuracy of the new BIO-VILCAD score but still significantly improved risk classification (NRI: 12.5%; P<0.001).

Conclusion: The VILCAD biomarker score based on routine parameters complemented by novel biomarkers outperforms previous risk algorithms and allows more accurate classification of stable CAD patients enabling physicians to choose more personalized treatment regimens for their patients.

The assessment of cardiovascular risk and the prevention of recurrent events in patients with established coronary artery disease (CAD) is a major public health issue (3). The main principle of preventive medicine is to initiate interventions appropriate to the level of the patient's individual risk. Therefore, thoughtful risk assessment is an indisputable element of preventive care. In stable CAD patients major cardiovascular events only occur with an estimated annual rate of 1 to 2% (3, 4). Therefore, in this patient population the estimation of the accumulating long-term risk is crucial in order to efficiently apply measures of secondary prevention (5). The VIenna and Ludwigshafen CAD (VILCAD) score was the first risk score specifically tailored for stable CAD patients encompassing routinely available clinical and laboratory variables (Goliasch et al., 2012; Eur Heart J). Nevertheless, numerous important novel biomarkers have not been evaluated in the developmental process of the VILCAD score (Goliasch et al, 2012; Eur Heart J).

Novel biomarkers have become an integral part in the assessment of patients with cardiovascular disease (3). These parameters cover different pathophysiological pathways of CAD development and have previously been implicated in an increased risk of cardiovascular disease and death. Prior studies demonstrated that simultaneous assessment of multiple biomarkers significantly enhances established cardiovascular risk prediction scores. However, no study has explored the possibility of refining cardiovascular risk estimation in stable CAD patients with the use of an extensive set of novel biomarkers.

The aim of our study was to further refine risk prediction in stable CAD by creating a new risk prediction model encompassing various pathophysiologic pathways. We therefore assessed the independent predictive power of 135 novel biomarkers from different pathophysiological pathways for long-term mortality in stable CAD patients and added these markers to the previously described VILCAD score, which is composed of routine parameters. In addition, we aimed to generate a risk score exclusively based on biomarkers, which would enable physicians to assess the long-term risk of stable CAD patients with a single blood draw.

**Materials and Methods**

Study cohort

The Ludwigshafen Risk and Cardiovascular Health (LURIC) study consists of 3,316 Caucasian patients admitted for coronary angiography between 1997 and 2000 to the Cardiac Center Ludwigshafen (Germany). The detailed protocol has been previously described (11). The study was approved by the ethics committee at the "Ärztekammer Rheinland-Pfalz" and was conducted in accordance with the "Declaration of Helsinki." Informed written consent was obtained from all participants. For the present analysis we selected a subgroup of 1275 LURIC participants with stable CAD.

Clinical definitions

CAD was assessed using coronary angiography and defined as previously described (Goliasch et al, 2012; Eur Heart J). Left ventricular ejection fraction (LVEF) was calculated using the Simpson method and categorized into normal (>55%), mildly reduced (45-54%), moderately reduced (30-44%) and severely reduced (< 30 %). Classic cardiovascular risk factors such as hypertension, diabetes, smoking, and lipid disorders were recorded as previously described (11).

Laboratory analyses

Venous blood was drawn in study participants after an overnight fasting period under standardized conditions. Within 30 min after venipuncture, the remaining blood was centrifuged at 3,000g for 10 min, immediately aliquoted and frozen at -80 °C until further analysis. A summary of analytic methods has been reported previously (11). Total cholesterol, high-density lipoprotein (HDL) cholesterol, and triglyceride concentrations were determined enzymatically. The LDL and very low-density lipoproteins (VLDL) were separated by ultracentrifugation at a density of 1.0063 kg/l; HDL cholesterol was obtained after precipitating LDL in the 1.0063 kg/l infranate with phosphotungstic acid/MgCl$_2$. NT-pro-BNP was measured by electro-chemiluminescence on an Elecsys 2010 (Roche Diagnostics). Inter-assay CVs were 3.2 % and 2.0 % at mean values of 157 and 5125 ng/L. Cystatin C was assayed by immunonephelometry (N-Latex Cystatin C, Dade Behring, Marburg, Germany) with intra-assay CVs of 1.5-3.1 % and inter-assay CVs of 1.5-3.5 %. Plasma renin concentration was determined by an immunoradiometric assay (Active Renin, Nichols Institute Diagnostics, Capistrano, USA). Intra-assay and inter-assay coefficients of variation were both < 10 %. Serum levels of 25OH vitamin D were measured using a radioimmunoassay (DiaSorin SA, Antony, France) with intra-assay and inter-assay coefficients of variation of 8.6 % and 9.2 %, respectively.

Study endpoints and follow-up

The primary study endpoint was all-cause mortality. Information on vital status was obtained from local registries. No patient was lost to follow-up. Death certificates were obtained in 97% of deceased participants and used to classify them into those who died of cardiovascular versus non-cardiovascular cause. This classification was performed independently by two experienced clinicians who were blinded to the study participants, except for information that was required to classify the cause of death.

Statistical analysis

Continuous data were presented as median and interquartile range, discrete data as counts and percentages. The Mann-Whitney-U test and chi-square test were used for comparisons between groups, as appropriate. Cox proportional hazard regression analysis was applied to assess the effect of variables on survival. Continuous variables underwent log transformation before entering analysis. A bootstrap resampling procedure was used to identify best-fitting variables for the multivariable Cox regression model. Before inclusion of variables into the bootstrapping procedure collinearity between biomarkers was assessed using Pearson correlation coefficients (data not shown). In case of close correlation only the strongest univariate predictor of a cluster (defined by the highest univariate hazard ratio for an increase of 1 standard deviation (1-SD)) entered the selection procedure. 100 repeats with forward selection and 100 repeats with backward selection (with a P <0.1 for selection) were performed. Variables selected in more than 80 % of all repeats were included in the final multivariable model. The discriminatory power of the designed risk scores was assessed using Harrell's C-statistic. For further analysis we stratified the cohort by tertiles of the biomarker risk scores into low, moderate and high risk. An improvement of individual risk prediction was examined by the net reclassification improvement (NRI) as described by Pencina et al. (13). Kaplan-Meier analysis (log-rank test) was applied to verify the time-dependent discriminative power of risk scores. Cut-off values for a simplified score using categorized variables were estimated using chi-square values derived from Martingale residuals of the Cox model for all possible cut-off values. For optimized clinical use these calculated cut-off values were rounded and extreme values were avoided. Two-sided P-values < 0.05 were used to indicate statistical significance. SPSS 18.0 (IBM SPSS, USA) and STATA 11 (StataCorp LP, USA) were used for all analyses.

**Results**

Baseline characteristics

We included 1275 stable CAD patients of the LURIC study. Of these study participants, 449 died (35%) during a median follow-up of 9.8 years (IQR 7.0-10.5), 284 of these deaths (63%) were due to cardiovascular events. The cause of death of 11 individuals was unknown. These patients were included in calculations of all-cause mortality (n=449) but not in calculations considering cardiovascular mortality. The study cohort included 962 male patients (75%). The median age was 65 years (IQR 58-71). Detailed baseline characteristics are shown in Supplementary Table 1.

Bootstrapping results and survival analysis

We tested a total of 135 biomarkers from the LURIC database for complementary inclusion in the original VILCAD score which consists of age, HbA1c, LVEF, serum cholinesterase, creatinine, and heart rate. Using bootstrapping age, HbA1c, LVEF, heart rate, Nt-proBNP, gender, renin, 25OH vitamin D and cystatin C were selected as significant predictors of mortality in the new biomarker model (Figure 4). From the variables of the original VILCAD score creatinine was dropped because of a close correlation with cystatin C. Furthermore, cholinesterase was excluded from the final model due to missing significance. The correlation of the remaining variables was only weak to modest. Pearson correlation coefficients for the selected (log transformed) variables are presented in Supplementary Table 3 with the highest coefficients being 0.47 between cystatin C and NT-proBNP, 0.34 between renin and age and 0.32 between NT-proBNP and age. All other correlation coefficients were <0.3. In the univariate Cox regression analysis, the strongest risk factors for mortality in this group of stable CAD patients were NT-proBNP with a HR for an 1-SD increase of 2.21 (95% CI: 2.00-2.43) and age with a HR of 1.84 (95%CI: 1.64-2.07) (Table 3). 25OH vitamin D3 showed a protective effect with a HR of 0.69 (95%CI: 0.63-0.76) per 1-SD increase. C-statistics for individual markers ranged from 0.58 (heart rate) to 0.71 (NT-proBNP, Table 3). In multivariate analysis adjusted for each variable in the final model NT-proBNP and age displayed the highest HRs with 1.63 (95%CI: 1.44-1.84) and 1.51 (95%CI: 1.34-1.70), respectively.

Risk score design and comparison with original VILCAD score

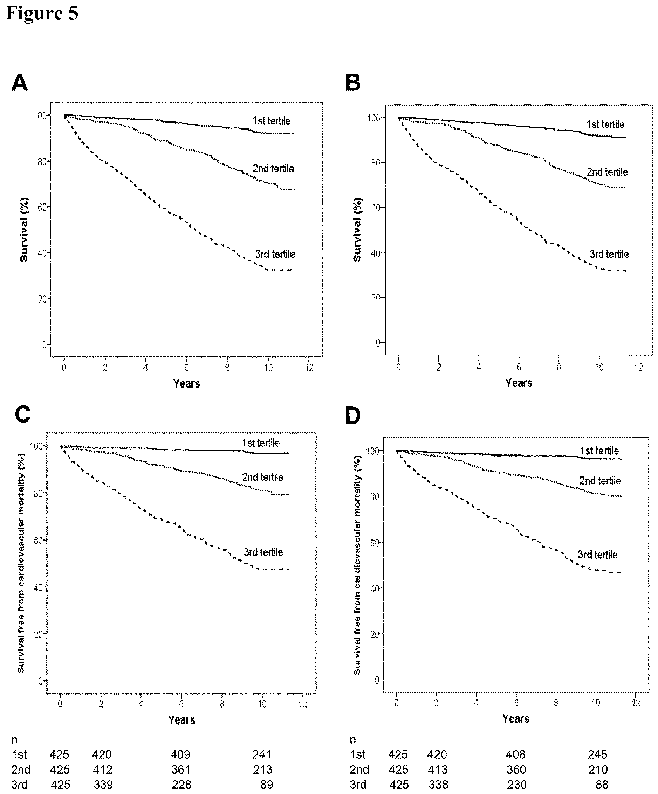

From the variables obtained by the bootstrapping procedure we calculated a weighted biomarker risk score for 10-year survival (Supplementary Method 3). The VILCAD biomarker score achieved a C-statistic of 0.78 which was significantly better than both the C-statistic of 0.73 for the original VILCAD score (p=0.035 for comparison) and the C-statistic of 0.71 for the best single marker NT-proBNP (p<0.001 for comparison). NRI analysis confirmed that individual risk prediction was improved by 14.9% (95%CI: 5.7-24.16, P<0.001) compared to the original VILCAD score (Table 4). The improvement of individual risk prediction was mainly driven by a downgrading of 34 % of patients without an event initially classified as moderate to high-risk patients and an upgrading of 43% of patients with an event initially classified as low to moderate-risk patients. Finally, we used Kaplan-Meier curves to analyze the discriminatory power of our biomarker scores by plotting tertiles of the respective scores for all-cause mortality as well as cardiovascular mortality (Figure 5). 10-year survival rates were 92 %, 70 % and 32 % for the first, second and third tertile (P< 0.001 between all tertiles), respectively. A comparable trend was observed for cardiovascular mortality with 10-year event-free rates of 97 %, 81 % and 47 % (P< 0.001 between all tertiles).

To address the secondary aim of a biomarker score using only laboratory data from a single blood draw (and demographics) another score without LVEF was tested. This BIO-VILCAD score achieved a C-statistic of 0.77 (p= 0.09 for comparison with original VILCAD score). Net individual risk prediction was significantly improved by 12.5 % (95 %CI: 5.2-19.9, P<0.001) compared with the original VILCAD score (Table 4B). Similarly to the VILCAD biomarker score the improvement in risk classification was driven by a downgrading of event-free patients originally classified as moderate to high risk and an upgrading of patients with events initially classified as low to moderate risk. For the BIO-VILCAD score 10-year survival rates were 92%, 70% and 33% for the first, second and third tertile, respectively (P<0.001 between all tertiles, Figure 5). With regard to cardiovascular mortality the 10-year event-free rates were 96%, 81% and 48%, respectively (P<0.001 between all tertiles).

Simplified score

Finally, a simplified VILCAD biomarker score was designed for optimal clinical use. Variables selected by bootstrapping were categorized using optimized cut-off values as defined in Figure 6. The strongest predictors age, LVEF and Nt-proBNP contributed 2 points, the remaining variables 1 point. The protective factor 25OH vitamin D contributed 1 point when its value was below the cut-off. The simplified VILCAD biomarker score still achieved a competitive predictive value with a C-statistic of 0.75 (p=0.001 for comparison with simplified VILCAD score). The simplified VILCAD biomarker score discriminated very well between low and high risk. The 10-year mortality ranged from 5.8% with zero points to 88.2% with seven or more points (Figure 6A). When excluding LVEF, the simplified BIO-VILCAD score still performed well with a C-statistic of 0.74 (p=0.028 for comparison with simplified VILCAD risk score). In accordance, the simplified BIO-VILCAD score showed a high spread of 10-year mortality from 7.5% with zero points to 89.8% with seven or more points (Figure 6B).

Discussion

We recently developed the VILCAD score using routinely available biomarkers for the risk prediction of stable CAD patients (Goliasch et al., 2012, Eur Heart J, 33: 2282-2289). Now we extended and refined our VILCAD risk score by the inclusion of new biochemical markers that can be easily measured with a single blood draw. The final set of nine variables was selected by a bootstrap resampling procedure from a large set of 135 clinical and biochemical markers representing various pathophysiological pathways. This comprehensive set of variables provided the opportunity to tailor a risk score precisely adapted to the risk profile of patients with stable CAD. From the six variables of the original VILCAD score age, LVEF, heart rate, and HbAlc remained in the new biomarker score. Nt-proBNP, sex, renin, 25OH vitamin D and cystatin C were added to the VILCAD biomarker score due to their consistent selection in the bootstrapping procedure. The new VILCAD biomarker score achieved a significantly improved discriminatory power with a C-statistic of 0.78 and a NRI of 14.9 % compared to the original VILCAD score. As LVEF, which was part of the original VILCAD score, might not be readily available in general practice we also tested a BIO-VILCAD score without this parameter. Exclusion of LVEF decreased the performance slightly but the BIO-VILCAD score was still significantly superior to the original VILCAD score with respect to improved risk classification with a NRI of 12.5%. Furthermore, both variants of the biomarker score significantly improved risk prediction compared to the strongest single biomarker NT-proBNP.

The only risk score for stable CAD patients that had been described in the literature before the VILCAD score was a score proposed by Marschner et al. (7) which achieved a C-statistic of 0.64 in the LURIC cohort. In parallel to the design of the VILCAD score, another score for patients with known cardiovascular disease has been developed using the REACH registry with an intermediate-term follow-up of 2 years (28). In accordance with the VILCAD score, cardiac failure and diabetes were part of this risk prediction model whereas age, heart rate, HbA1c, creatinine and cholinesterase were not included. On the other hand, smoking, body mass index and history of cardiovascular disease were included in the REACH score but did not qualify for the final model of the VILCAD score in the bootstrap resampling procedure. It should be noted however that only 72% of the REACH patients used to generate and validate that score suffered from CAD whereas the remaining 28% presented with cerebrovascular or peripheral artery disease, which is in contrast to our patients who all had angiographically proven CAD.

Biomarker scores may be even more efficient in clinical practice as only a single blood draw is required for risk estimation and additional disease pathways may be taken account of. However, increased costs for the measurement of biomarkers need to be justified by an improvement in the accuracy of risk prediction. So far, only one biomarker score for stable CAD has been published by Schnabel et al. (21). This biomarker score included Nt-proBNP, growth differentiation factor 15 (GDF-15), mid-regional pro-adrenomedullin (MR-proADM), cystatin C and midregional pro-atrial natriuretic peptide (MR-proANP) in the final selection. However, this multi-biomarker score showed an only moderate C-statistic of 0.69 for cardiovascular mortality in the AtheroGene cohort with a median follow-up of 3.6 years which did not exceed the C-statistic of the best single biomarker NT-proBNP. One may speculate that collinearity, particularly due to a close correlation between the neurohormones Nt-proBNP, MR-proADM and MR-proANP, may have impeded a higher predictive value of the score.

In comparison the VILCAD biomarker score achieved a C-statistic of 0.78 for a more challenging long-term follow-up of almost 10 years in the LURIC cohort with fairly similar baseline characteristics. A potential explanation for the difference in predictive accuracy may be that the VILCAD biomarker score was based on an initial set of more than 100 biomarkers compared with 12 biomarkers in the AtheroGene cohort. While Nt-proBNP and cystatin C were included in both scores the VILCAD biomarker score considered pathways which were not available in the AtheroGene cohort and additionally included age, LVEF, heart rate, HbA1c, sex, renin and 25OH vitamin D in the final model. On the other hand, the score described by Schnabel et al. (21) additionally included GDF-15, MR-proANP and MR-proADM.

All biochemical markers that have been included into the VILCAD biomarker score have long been recognized as risk markers for morbidity and mortality. Both brain natriuretic peptide (BNP) and the N-terminal (NT) fragment of pro-BNP are well-established biomarkers used for the diagnosis of heart failure and have been investigated extensively in patients with acute coronary syndrome and myocardial infarction (29, 30). In LURIC as well as in several other studies their utility as risk markers in patients with stable CAD has been shown. Renin is part of the renin-angiotensin-aldosterone system (RAAS), involved in the pathogenesis of a number of hypertensive disorders and might contribute to the development and progression of cardiovascular disease. Renin has been associated with total mortality in the Framingham Offspring Study whereas no association with cardiovascular events or CVD was found (31, 32). Recently, Tomaschitz et al. reported a significant association of renin with CVD in LURIC (33). Vitamin D deficiency is very common worldwide. Besides the well recognized importance of vitamin D for calcium homeostasis and skeletal health a role in a number of extraskeletal pathological conditions ranging from infection to chronic diseases and cancer has been proposed. Recent meta-analyses of randomized controlled trials reported an association of vitamin D supplementation with decreased total mortality. In LURIC, low levels of 25OH vitamin D have been associated with diabetes, stroke, inflammation, total and cardiovascular mortality (34). Cystatin C represents an alternative endogenous surrogate parameter for estimating glomerular filtration rate (eGFR). There is evidence that eGFR equations that incorporate cystatin C concentration alone or in combination with creatinine may perform better in the normal GFR range than those relying solely on creatinine and a number of studies have shown that cystatin C is a better and more linear predictor of mortality and cardiovascular events than creatinine although this has not been confirmed in all populations.

Several studies have shown that a multi-marker score is superior with regard to risk prediction (21, 26). However, the clinical use of multi-marker scores is limited, mainly due to time constraints. A simplified risk score using categorized variables with optimized cut-off may help to overcome this limitation while it still provides accurate risk prediction as demonstrated with the simplified VILCAD biomarker score. To further facilitate the clinical use, a simplified BIO-VILCAD score without LVEF was calculated, which allows to accurately predict the risk with a single blood draw followed by automated calculation of risk in the clinical laboratory. While risk prediction tools have been previously scrutinized for their moderate relevance for personalized treatment decisions new statistical tools such as the net reclassification index provide information about the percentage of persons who benefit from improved risk prediction. A large part of the improvement in reclassification with the simplified biomarker score was derived from correctly downgrading the risk level of event-free individuals. Though, there was an even large percentage of patients with events who were correctly reclassified into a higher risk category. Almost half of these patients that were classified as "low risk" by the original VILCAD score were upgraded to "moderate risk" by the biomarker score and about one third of these patients classified as "moderate risk" by the original score was correctly upgraded into the "high risk" group. This improvement, both for patients that do not experience an event as well as for patients that suffer an event, allows to better tailor the individual treatment option preventing patients with low risk to be over-treated and, even more important, helping to identify patients that need intensive therapy because of high individual risk.

Limitations: The specific distribution of low, moderate, and high-risk individuals in a cohort can have an effect on how well a panel of biomarkers works. Furthermore, our patients were recruited between 1997 and 2000 and since then strategies for secondary prevention have been modified which might affect the performance of our score in today's practice. Novel biomarkers have been measured only once from blood samples which had been collected at baseline and frozen at -80°C until measurement but the included biomarkers are all expected to be relatively stable under these conditions. The newly included biomarkers raise the costs associated with the score but on the other hand all markers can be easily measured from a single blood sample.

A strength of our study is the large and homogenous cohort of stable CAD patients with complete follow-up over a median of 9.8 years. During the long follow-up period we recorded a number of 449 deaths, which gives us sufficient power to detect even moderate effect sizes of individual markers. Another strength is the excellent clinical and biochemical characterization of our patients, which allowed us to compare a very large number of biomarkers and to select the ones with the largest effect sizes and little collinearity.

Conclusions: The VILCAD biomarker score encompassing nine variables representing different pathophysiological pathways (heart function: heart rate, LVEF and NT-proBNP; kidney function: cystatin C; glucose metabolism: HbA1c; blood pressure: renin; calcium homeostasis and immune function: vitamin D) as well as age and sex has an excellent discriminatory power to classify stable CAD patients into risk groups which is even superior to the original VILCAD score. All variables except LVEF are easily measurable and omission of LVEF decreases the performance only slightly. Therefore, the VILCAD biomarker score represents a valuable and easy to use tool for risk prediction of stable CAD patients in the clinical routine.

**Table 3: Unadjusted and adjusted effects on mortality**

Cox proportional hazard model of variables selected by bootstrapping. Hazard ratios (HR) refer to a 1-SD increase in continuous variables and to a reduction of one category of LVEF. HRs are adjusted (adj.) for all variables selected by bootstrapping i.e. for sex, age, heart rate, LVEF, pro-BNP, HbA1c, renin, 25-OH vitamin $D_3$, and cystatin C. The discriminatory power of the respective variables was assessed using Harrell's C-statistic. SD - standard deviation.

| Variables | SD | Crude HR (95%CI) | P-value | Adj. HR (95%CI) | P-value | C-statistic |
|---|---|---|---|---|---|---|
| Male | ---- | 1.28 (1.02-1.61) | **0.033** | 1.63 (1.28-2.08) | **<0.001** | / |
| Age | 11.74 | 1.84 (1.64-2.07) | **<0.001** | 1.521(1.34-1.70) | **<0.001** | 0.65 |
| Heart rate | 11.8 | 1.30 (1.18-1.43) | **<0.001** | 1.12 (1.01-1.23) | **0.026** | 0.58 |
| LVEF | ---- | 1.75 (1.60-1.91) | **<0.001** | 1.20 (1.08-1.33) | **0.001** | 0.63 |
| Nt-proBNP | 2042.6 8 | 2.21 (2.00-2.43) | **<0.001** | 1.63 (1.44-1.84) | **<0.001** | 0.71 |
| HbA1c | 1.30 | 1.43 (1.32-1.55) | **<0.001** | 1.26 (1.15-1.38) | **<0.001** | 0.60 |
| Renin | 332.01 | 1.42 (1.30-1.54) | **<0.001** | 1.24 (1.13-1.36) | **<0.001** | 0.59 |
| 25-OH vitamin $D_3$ | 9.27 | 0.69 (0.63-0.76) | **<0.001** | 0.75 (0.68-0.83) | **<0.001** | 0.60 |
| Cystatin C | 0.47 | 1.46 (1.38-1.54) | **<0.001** | 1.18 (1.08-1.29) | **<0.001** | 0.66 |

**Table 4: Reclassification table** - Reclassification table comparing the weighted biomarker score (A) with or (B) without LVEF to the weighted VILCAD risk score.

| A | | | | | |
|---|---|---|---|---|---|
| | VILCAD | Biomarker | | | |
| | Risk class | Low | Moderate | High | Total |
| No event (n=826) | Low | 78% (280) | 21% (76) | 1% (4) | 100% (360) |
| | Moderate | 33% (102) | 55% (169) | 13% (40) | 100% (308) |
| | High | 4% (6) | 33% (52) | 63% (100) | 100% (158) |
| Event (n=449) | Low | 40% (26) | 49% (32) | 11% (7) | 100% (65) |
| | Moderate | 5% (6) | 61% (71) | 34% (40) | 100% (117) |
| | High | 0% (0) | 1 0% (27) | 90% (240) | 1 00% (267) |
| B | | | | | |
| | VILCAD | Biomarker without LVEF | | | |
| | Risk class | Low | Moderate | High | Total |
| No event (n=826) | Low | 76% (274) | 22% (79) | 2% (7) | 100% (360) |
| | Moderate | 34% (105) | 53% (163) | 13% (40) | 100% (308) |
| | High | 6% (9) | 36% (57) | 58% (92) | 100% (158) |
| Event (n=449) | Low | 40% (26) | 47% (31) | 13% (8) | 100% (65) |
| | Moderate | 8% (9) | 55% (65) | 37% (43) | 100% (117) |
| | High | 0% (0) | 13% (35) | 87% (232) | 100% (267) |
| Data are given as % row (n). | | | | | |

**Supplemental Table 2: Baseline characteristics and biomarkers** - Continuous variables are given as median (interquartile range [IQR]). Left ventricular ejection fraction (LVEF); ECG abnormalities are defined as ST-segment deviation and/or T wave inversion; bpm beats per minute

| | LURIC study population (n=1275) |
|---|---|
| Age, years | 65 (58- 71) |
| Male gender, n (%) | 962 (75%) |
| BMI, kg/m$^2$ | 27.2 (24.7- 29.8) |
| Multi-vessel-disease, n (%) | 745 (58%) |
| Systolic blood pressure, mmHg | 145 (128- 161) |
| Heart rate, bpm | 68 (60- 77) |
| LVEF | |
| ≥55%, n (%) | 778 (61%) |
| 45-54%, n (%) | 207 (16%) |
| 30-44%, n (%) | 177 (14%) |
| <30%, n (%) | 68 (5%) |
| ECG abnormalities, n (%) Medication | 351 (28%) |
| Platelet inhibitors, n (%) | 1252 (98%) |
| Vitamin K antagonists n (%) | 101 (07%) |
| Beta blocker, n (%) | 778 (61%) |
| ACE inhibitors/Angiotensin II receptor blockers, n (%) | 727 (57%) |
| Digitalis, n (%) | 217 (17%) |
| Nitrates, n (%) | 359 (28%) |

(continued)

| | LURIC study population (n=1275) |
|---|---|
| Statins, n (%) | 624 (49%) |
| Hypertension, n (%) | 807 (63%) |
| Current smokers, n (%) | 192 (15%) |
| Family history CAD, n (%) | 456 (36%) |
| Previous stroke, n (%) | 132 (10%) |
| Diabetes, n (%) | 451 (35%) |
| Hbalc, mg/dl | 6.1 (5.7- 6.8) |
| Glucose, mg/dl | 94 (84- 111) |
| Hypercholesterolemia, n (%) | 848 (67%) |
| Total cholesterol, mg/dl | 207 (180- 237) |
| VLDL cholesterol, mg/dl | 31 (21- 46) |
| LDL cholesterol, mg/dl | 114 (93- 138) |
| HDL cholesterol, mg/dl | 37 (32- 45) |
| VLDL triglycerides, mg/dl | 101 (66- 152) |
| LDL triglycerides, mg/dl | 29 (23- 36) |
| HDL triglycerides, mg/dl | 14 (11- 19) |
| Lp (a), mg/dl | 16.2 (7-41) |
| ApoA-I, mg/dl | 128 (114- 146) |
| ApoA-II, mg/dl | 41.6 (36.0- 47.6) |
| Average LDL particle radius, nm | 8.26 (8.13- 8.37) |
| Hypertriglyceridemia, n (%) | 596 (47%) |
| Triglycerides, mg/dl | 144 (108- 198) |
| Free fatty acids, mmol/L | 0.60 (0.43- 0.85) |
| Zinc, $\mu$mol/ L | 87 (78- 96) |
| Iron, $\mu$g/dl | 91 (70- 115) |
| Sodium, mmol/L | 141 (139- 143) |
| Copper, $\mu$g/dl | 105 (91- 121) |
| Coeruloplasmin, mg/dl | 29 (25- 32) |
| Soluble transferrin receptor, mg/L | 1.28 (1.07- 1.57) |
| Phosphate, mg/dl | 3.5 (3.1- 3.8) |
| Beta-crosslaps, ng/ml | 0.31 (0.21-0.44) |
| Creatinine, mg/dl | 0.9 (0.8- 1.1) |
| Cystatin C, mg/L | 0.9 (0.8- 1.1) |
| Renin, pg/mL | 20 (10- 45) |
| Angiotensin I | 1470 (1160- 1881) |
| Angiotensinogen, nmol/L | 1135 (897- 1451) |
| Uric acid, mg/dl | 4.9 (4.1- 6.0) |
| Protein, g/l | 6.9 (6.6- 7.3) |
| Albumin, g/l | 44.0 (41.0- 48.0) |
| Vitamin B6, $\mu$g/L | 9.2 (5.7- 14.4) |
| 25OH-vitamin $D_3$, $\mu$g/L | 15.3 (9.9- 22.8) |
| Cholinesterase, kU/L | 5.81 (4.94- 6.68) |
| Alkaline phosphatase, U/L | 116 (96- 138) |
| Noradrenalin, ng/L | 336.5 (236.- 472.3) |
| 17$\beta$ estradiol, ng/L | 37 (28- 47) |
| Lactate dehydrogenase, U/L | 166 (146- 187) |
| Parathyroid hormone, pg/ml | 31.0 (23.0- 41.8) |
| Lycopin, $\mu$mol/L | 0.25 (0.15- 0.39) |
| Homocysteine, $\mu$mol/L | 12.5 (10.0- 15.7) |

(continued)

| | LURIC study population (n=1275) |
|---|---|
| LpPLA2, U/L | 471 (396- 556) |
| IL-6, ng/L | 3.1 (1.8- 5.7) |
| C-reactive protein, mg/dl | 0.36 (0.09- 0.79) |
| Myeloperoxidase, $\mu$g/L | 30.2 (21.0- 48.8) |
| aPC ratio | 4.4 (4.1- 4.9) |
| APTT, sec | 33.0 (31.0- 36.0) |
| Antithrombin III, % | 98 (89- 106) |
| INR quick | 1.04 (0.99- 1.10) |
| Factor VII, U/dl | 122 (106- 136) |
| Thrombomodulin, $\mu$g/L | 47 (36- 60) |
| vWF, U/dl | 156 (121- 197) |
| D-dimer, mg/L | 0.36 (0.22- 0.60) |
| Tissue factor pathway inhibitor, $\mu$g/L | 1.23 (1.02- 1.44) |
| PAI-1 antigen, $\mu$g/L | 25.7 (16.5- 38.0) |
| tPA antigen, $\mu$g/L | 11.7 (9.4- 14.8) |
| Platelets, 1000/$\mu$l | 218 (182- 262) |
| Mean platelet volume, fl | 9.0 (8.3- 9.6) |
| Erythrocytes, $10^{12}$/L | 4.6 (4.3- 4.9) |
| Leukocytes, 1000/$\mu$l | 6.72 (5.65- 8.1) |
| fT3, pmol/L | 4.8 (4.3- 5.4) |
| TnT, pg/ml | 10.2 (6.0- 19.3) |
| Copeptin, pmol/L | 6.9 (4.3- 12.1) |
| Adiponectin, $\mu$g/ml | 8.4 (5.7- 12.6) |
| Neopterin, nmol/L | 5.7 (4.6- 7.3) |
| NT-proBNP, ng/L | 302 (116- 922) |

**Supplemental Table 3: Correlation table of continuous variables in the multivariate model** - Log-transformed variables are correlated using Pearson's correlation coefficient.

| Variables | Age | Heart rate | Pro-BNP | Hbalc | Renin | 25-OH vitamin $D_3$ |
|---|---|---|---|---|---|---|
| Heart rate | r=-0.01 p=0.73 | ------ | ------ | ------ | ------ | ------ |
| Pro-BNP | r=0.32 **P<0.001** | r=0.07 **P=0.02** | ------ | ------ | ------ | ------ |
| HbA1c | i=0.14 **P<0.001** | r=0.13 **P<0.001** | i=0.14 **P<0.001** | ------ | ------ | ------ |
| Renin | r=-0.03 P=0.34 | r=0.22 **P<0.001** | r=0.06 P=0.05 | r=0.15 **P<0.001** | ------ | ------ |
| 25-OH vitamin $D_3$ | r=0.12 **P<0.001** | r=-0.09 **P<0.001** | r=-0.16 **P<0.001** | r=-0.07 **P=0.02** | r=-0.07 **P=0.02** | ------ |
| Cystatin C | r=0.26 **P<0.001** | r=0.11 P=0.70 | r=0.47 **P<0.001** | r=0.09 **P=0.001** | r=-0.12 **P<0.001** | r=-0.12 **P<0.001** |

**Supplemental Method 3:** Generation of the weighted VILCAD biomarker score

**General:**

Weighted risk score = [V1 – mean of V1] * beta coefficient of V1 + [V2 – mean of V2] * beta coefficient of V2 + [V3 – mean of V3] * beta coefficient of V3 + ……..+ [Vn – mean of Vn] * beta coefficient of Vn.

**Weighted VILCAD biomarker score:**

= [age-4.151] * 2.69+ [HbA1c - 1.848] * 1.320 + [heart rate - 4.221] * 0.633 + LVEF * 0.182 + [proBNP - 5.786] * 0.345 + female * -0.495 + [renin - 3.168] * 0.172 + [25-hydroxy vitamin D - 2.688] * -0.492 + [cystatin C - 0.019] * 0.593

Beta coefficients were derived from a multivariable Cox regression model including all variables of the respective model. Continuous variables were log-transformed before inclusion in the score; n, number of variables of the respective risk score; V, variable

**Cited references**

(1) Bassand J.P. et al., 2007. Eur Heart J, 28: 1598-1660.
(2) Fuster V. et al., 1992. N Engl J Med, 326: 242-250.
(3) Morrow D.A., 2010. Circulation, 121: 2681-2691.
(4) Steg P.G. et al., 2007. JAMA, 297: 1197-1206.
(5) Graham I. et al., 2007. Eur Heart J, 28: 2375-2414.
(6) Clark A.M. et al., 2005. Ann Intern Med, 143: 659-672.
(7) Marschner I.C. et al., 2001. J Am Coll Cardiol, 38: 56-63.
(8) Manjunath G. et al., 2003 J Am Coll Cardiol, 41: 47-55.
(9) Sachdev M. et al., 2004, J Am Coll Cardiol 43: 576-582.
(10) Niessner A. et al., 2005. Thromb Haemost, 93:949-954.
(11) Winkelmann B.R. et al., 2001.Pharmacogenomics, 2:S1-73.
(12) Tomaschitz A. et al., 2010. Eur Heart J, 31:1237-1247.
(13) Pencina M.J. et al., 2008. Stat Med, 27:157-172; discussion 207-112.
(14) Granger C.B. et al., 2003. Arch Intern Med, 163: 2345-2353.
(15) Hemingway H. et al., 2009. BMJ, 339: b4184.
(16) Yan A.T. et al., 2007. Eur Heart J, 28: 1072-1078.
(17) Libby P., 1995. Circulation, 91: 2844-2850.
(18) Naghavi M. et al., 2003. Part I. Circulation, 108: 1664-1672.
(19) Bogaty P. et al., 1993. Circulation, 87: 1938-1946.
(20) Vranckx P. et al., 2011. EuroIntervention 7: 859-871.
(21) Schnabel R.B. et al., 2010. Eur Heart J 31: 3024-3031.
(22) Emond M. et al. 1994. Circulation 90: 2645-2657.
(23) Mock M.B. et al., 1982. Circulation 66: 562-568.
(24) Calderon-Margalit R. et al., 2006. Clin Chem 52:845-852.
(25) Goliasch G. et al., 2012. Clin Chem Jan 31. (Epub ahead of print)
(26) Wang T.J., 2010. J Am Coll Cardiol 55:2092-2095.
(27) Kooter A.J. et al., 2011. Circulation, 124: 741-745.
(28) Wilson P.W. Am J Med 2012; 125: 695-703.
(29) Omland T., Circulation 2002; 106: 2913-8.
(30) de Lemos J.A., N Engl J Med 2001; 345: 1014-21.
(31) Wang T.J., N Engl J Med 2006; 355: 2631-9.
(32) Parikh N.I., Eur Heart J 2007; 28: 2644-52.
(33) Tomaschitz A., Eur Heart J 2011, 32: 2642-9.
(34) Thomas G.N., Diabetes Care 2012; 35: 1158-64.

**Claims**

1. An in vitro method for determining the risk of mortality in a patient with a cardiovascular disease, wherein the cardiovascular disease is a stable coronary artery disease, comprising the steps of

a) analysing a set of biological parameters obtained from the patient, wherein the set of biological parameters comprises the following variables:

the patient's gender,
the patient's age,
at least one indicator of myocardial function or myocardial performance, wherein the at least one indicator of myocardial function or myocardial performance is defined by the patient's left ventricular ejection fraction (LVEF) in % of volume per volume, or by the patient's blood concentration of N-terminal pro-B-type natriuretic peptide (NT-proBNP),
at least two indicators of renal function or renal performance, wherein the at least two indicators of renal function or renal performance are defined by the patient's blood concentration of renin and cystatin C,
the patient's heart rate,
at least one indicator of blood glucose metabolism, wherein the at least one indicator of blood glucose metabolism is defined by the patient's blood concentration of glycated haemoglobin HbA1c in % of total haemoglobin,
and at least one indicator of blood calcium metabolism, wherein the at least one indicator of blood calcium metabolism is defined by the patient's blood concentration of 25(OH)-vitamin $D_3$,

b) setting the biological parameters into correlation, thereby providing a mortality risk score, wherein the risk score is indicative for mortality of the patient within the next 10 years, and wherein the risk score is indicative for a treatment of secondary prevention of the patient to reduce the patient's risk of mortality due to the cardiovascular disease, **characterized in that**
the mortality risk score is a simplified risk score $X_{1\#}$ and the biological parameters are set into correlation by a sum of at least eight quantities, the sum thereby providing the simplified risk score $X_{1\#}$,
wherein a first quantity is equal to 2 if the patient's age is equal to or more than 75 years and otherwise equal to 0,
wherein a second quantity is equal to 1 if the concentration of N-terminal pro-B-type natriuretic peptide (NT-proBNP) is equal to or more than 400 ng/L, equal to 2 if the concentration of N-terminal pro-B-type natriuretic peptide (NT-proBNP) is equal to or more than 2000 ng/L and otherwise equal to 0,
wherein a third quantity is equal to 1 if the concentration of glycated haemoglobin HbA1c is equal to or more than 6.5 % for patients with known diabetes and otherwise equal to 0,
wherein a fourth quantity is equal to 1 if the concentration of renin is equal to or more than 50 pg/mL and otherwise equal to 0,
wherein a fifth quantity is equal to 1 if the concentration of cystatin C is equal to or more than 1.2 mg/L or otherwise equal to 0,
wherein a sixth quantity is equal to 1 if the concentration of of 25(OH) vitamin $D_3$ is equal to or less than 10 ng/L and otherwise equal to 0,
wherein a seventh quantity is equal to 1 if the patient's heart rate is equal to or more than 75 beats per minute and otherwise equal to 0, and
wherein a eighth quantity is equal to 1 if the patient's gender is male and otherwise equal to 0,
wherein a simplified risk score $X_{1\#}$ of
equal to 0 or equal to 1 indicates a low risk of mortality,
equal to 2 or equal to 3 indicates a moderate risk of mortality,
equal to or more than 4 indicates a high risk of mortality,
wherein a low risk of mortality means a risk of mortality of below 20 %, a moderate risk of mortality means a risk of mortality of between 30 to 40 %, and a high risk of mortality means a risk of mortality of at least 50 %.

2. The method of claim 1, wherein the set of biological parameters comprises a further variable in form of an indicator of myocardial function or myocardial performance, wherein the indicator of myocardial function or myocardial performance is defined by the patient's left ventricular ejection fraction (LVEF) in % of volume per volume, and wherein the biological parameters are set into correlation by a sum of at least ninth quantities, the sum thereby providing the simplified risk score $X_{1a\#}$, preferably
wherein the ninth quantity is defined by the patient's left ventricular ejection fraction (LVEF) in % of volume per volume and wherein the ninth quantity is
equal to 1 if the left ventricular ejection fraction (LVEF) is moderately reduced, equal to 2 if the left ventricular ejection fraction is severely reduced and otherwise equal to 0,
wherein a moderately reduced left ventricular ejection fraction (LVEF) is defined by a value of 30 - 44 in % of volume per volume, and wherein a severely reduced left ventricular ejection fraction (LVEF) is defined by a value of below 30 in % of volume per volume,

wherein a simplified risk score $X_{1a\#}$ of

equal to 0 or equal to 1 indicates a low risk of mortality,

equal to 2 or equal to 3 indicates a moderate risk of mortality,

equal to or more than 4 indicates a high risk of mortality,

wherein a low risk of mortality means a risk of mortality of below 20 %, a moderate risk of mortality means a risk of mortality of between 30 to 40 %, and a high risk of mortality means a risk of mortality of at least 50 %.

3. The method of claim 1, wherein the risk score is a weighted risk score $X_{2\#}$ and the biological parameters are set into correlation by using the following formula, thereby providing the weighted risk score $X_{2\#}$:

$$X_{2\#} = (\text{variable } 1 - 4.151) * 2.69 + (\text{variable } 2 - 1.848) * 1.32 + (\text{variable } 3 - 4.221) * 0.633 + \text{variable } 4 * 0.182 + (\text{variable } 5 - 5.786) * 0.345 + (\text{variable } 6) * -0.495 + (\text{variable } 7 - 3.168) * 0.172 + (\text{variable } 8 - 2.688) * -0.492 + (\text{variable } 9 - 0.019) * 0.593,$$

wherein

a) variable 1 is defined by the log value of the patient's age in years,

b) variable 2 is defined by the log value of the patient's blood concentration of glycated haemoglobin HbAlc in % of total haemoglobin,

c) variable 3 is defined by the log value of the patient's heart rate in beats per minute,

d) variable 4 is defined as equal to 1 if the patient's left ventricular ejection fraction (LVEF) is moderately reduced, equal to 2 if the left ventricular ejection fraction (LVEF) is severely reduced and otherwise equal to 0, wherein a moderately reduced left ventricular ejection fraction is defined by a value of 30 - 44 in % of volume per volume, and wherein a severely reduced left ventricular ejection fraction is defined by a value of below 30 in % of volume per volume,

e) variable 5 is defined by the log value of the patient's blood concentration of N-terminal pro-B-type natriuretic peptide (NT-proBNP) in ng/L,

f) variable 6 is defined as equal to 1 if the patient's gender is male and equal to 0 if the patient's gender is female,

g) variable 7 is defined by the log value of the patient's blood concentration of renin in pg/ml,

h) variable 8 is defined by the log value of the patient's blood concentration of 25(OH) vitamin $D_3$ in ng/L,

i) variable 9 is defined by the log value of the patient's blood concentration of cystatin C in mg/L,

preferably wherein a weighted risk score $X_{2\#}$

in the range of less than or equal to 0 indicates a low risk of mortality,

in the range of 0 to 1 indicates an intermediate risk of mortality, and

in the range of more than or equal to 1 indicates a high risk or mortality, wherein a low risk of mortality means a risk of mortality of below 20 %, a moderate risk of mortality means a risk of mortality of between 30 to 40 %, and a high risk of mortality means a risk of mortality of at least 50 %.

4. An in vitro method for determining the risk of mortality in a patient with a cardiovascular disease, wherein the cardiovascular disease is a stable coronary artery disease, comprising the steps of

a) analysing a set of biological parameters obtained from the patient, wherein the set of biological parameters comprises at least the following variables: the patient's age, an indicator of myocardial performance, an indicator of renal performance, the patient's heart rate, an indicator of blood glucose metabolism,

b) setting the biological parameters into correlation, thereby providing a mortality risk score, wherein the risk score is indicative for mortality of the patient within the next 10 years, and wherein the risk score is indicative for a treatment of secondary prevention of the patient to reduce the patient's risk of mortality due to the cardiovascular disease,

wherein

i) the indicator of myocardial performance is defined by the patient's left ventricular ejection fraction in % of volume per volume,

ii) the indicator of renal performance is defined by the patient's blood concentration of serum creatinine,

iii) the indicator of blood glucose metabolism is defined by the patient's blood concentration of glycated haemoglobin HbAlc in % of total haemoglobin, and

wherein the mortality risk score is a simplified risk score $X_{1a}$ and wherein the biological parameters are set into correlation by a sum of at least six quantities, the sum thereby providing the simplified risk score $X_{1a}$, wherein a first quantity is equal to 2 if the patient's age is equal to or more than 75 years and otherwise equal to 0, wherein a second quantity is equal to 1 if the left ventricular ejection fraction is moderately reduced, equal to 2 if the left ventricular ejection fraction is severely reduced and otherwise equal to 0, wherein a moderately reduced left ventricular ejection fraction is defined by a value of 30 - 44 in % of volume per volume, and wherein a severely reduced left ventricular ejection fraction is defined by a value of below 30 in % of volume per volume, wherein a third quantity is equal to 1 if the concentration of glycated haemoglobin HbAlc is equal to or more than 6.5 % for patients with known diabetes and otherwise equal to 0, wherein a fourth quantity is equal to 1 if the serum creatinine concentration is equal to or more than 1.3 mg/dl and otherwise equal to 0, and wherein a fifth quantity is equal to 1 if the patient's heart rate is equal to or more than 75 beats per minute and otherwise equal to 0, wherein the set of biological parameters further comprises a variable in form of an indicator of liver performance, wherein the indicator of liver performance is defined by the patient's blood concentration of serum cholinesterase and wherein a sixth quantity is set to 1 if the concentration of serum cholinesterase is equal to or less than 4.4 Units/ml and otherwise equal to 0, wherein a simplified risk score $X_{1a}$ of equal to 0 indicates a low risk of mortality, equal to 1 or equal to 2 indicates an intermediate risk of mortality, equal to or more than 3 indicates a high risk of mortality, wherein a low risk of mortality means a risk of mortality of below 20 %, an intermediate risk of mortality means a risk of mortality of between 30 to 50 %, and a high risk of mortality means a risk of mortality of at least 60 %.

5. The method of claim 4, wherein the risk score is a weighted risk score $X_2$ and the biological parameters are set into correlation by using the following formula, thereby providing the weighted risk score $X_2$:

$$X_2 = (\text{variable } 1 - 4.14) * 2.33 + (\text{variable } 2) * 0.42 + (\text{variable } 3 - 1.73) * -1.05 + (\text{variable } 4 - 0.68) * 1.27 + (\text{variable } 5 - 4.21) * 1.47 + (\text{variable } 6 - 1.85) * 1.79,$$

wherein

a) variable 1 is defined by the log value of the patient's age in years,
b) variable 2 is defined as equal to 1 if the left ventricular ejection fraction is moderately reduced, equal to 2 if the left ventricular ejection fraction is severely reduced and otherwise equal to 0, wherein a moderately reduced left ventricular ejection fraction is defined by a value of 30 - 44 in % of volume per volume, and wherein a severely reduced left ventricular ejection fraction is defined by a value of below 30 in % of volume per volume,
c) variable 3 is defined by the log value of the patient's blood concentration of serum cholinesterase in Units/ml,
d) variable 4 is defined by the log value of the patient's blood concentration of serum creatinine in mg/dl,
e) variable 5 is defined by the log value of the patient's heart rate in beats per minute,
f) variable 6 is defined by the log value of the patient's blood concentration of glycated haemoglobin HbAlc in % of total haemoglobin,

preferably wherein a weighted risk score $X_2$ in the range of less than or equal to 0 indicates a low risk of mortality, in the range of 0 to 1 indicates an intermediate risk of mortality, and in the range of more than or equal to 1 indicates a high risk or mortality, wherein a low risk of mortality means a risk of mortality of below 20 %, a moderate risk of mortality means a risk of mortality of between 30 to 40 %, and a high risk of mortality means a risk of mortality of at least 50 %.

6. The method of any of the preceding claims, wherein the treatment of secondary prevention is in form of administered drugs, wherein the drugs are for treating hyperlipidaemia, regulating blood pressure, modifying the heart rate and/or for providing glycaemic control in diabetic patients, preferably wherein the drugs are for reducing the patient's blood concentration of cholesterol, for reducing the patient's blood pressure, and/or for reducing the patient's blood glucose concentration.

7. The method of any of the preceding claims, wherein the risk score is indicative for a treatment of secondary prevention of patients with an intermediate or moderate risk of mortality, or of patients with a high risk of mortality.

8. A computer program product directly loadable into the internal memory of a digital computer, comprising software code portions for implementing the calculation of the mortality risk score according to any one of claims 1 to 5 when said product is run on a computer, preferably comprising software code portions for implementing the calculation of the weighted risk score as defined in claim 3 or claim 5.

**Patentansprüche**

1. In vitro-Verfahren zum Bestimmen des Mortalitätsrisikos in einem Patienten mit einer kardiovaskulären Erkrankung, wobei die kardiovaskuläre Erkrankung eine stabile koronare Arterienerkrankung ist, umfassend die Schritte von:

   a) Analysieren eines Satzes von biologischen Parametern, die von dem Patienten erhalten werden, wobei der Satz von biologischen Parametern die folgenden Variablen umfasst:

   das Geschlecht des Patienten,
   das Alter des Patienten,
   mindestens einen Indikator von myokardialer Funktion oder myokardialer Leistung, wobei der mindestens eine Indikator von myokardialer Funktion oder myokardialer Leistung durch die linke ventrikuläre Ejektionsfraktion (LVEF) des Patienten in Volumen pro Volumen-% oder durch die Blutkonzentration von N-terminalem pro-B-Typ-natriuretischem Peptid (NT-proBNP) des Patienten definiert ist,
   mindestens zwei Indikatoren von Nierenfunktion oder Nierenleistung, wobei die mindestens zwei Indikatoren von Nierenfunktion oder Nierenleistung durch die Blutkonzentration von Renin und Cystatin C des Patienten definiert sind,
   die Herzfrequenz des Patienten,
   mindestens einen Indikator von Blutglukosemetabolismus, wobei der mindestens eine Indikator von Blutglukosemetabolismus durch die Blutkonzentration von glykiertem Hämoglobin HbA1c des Patienten in % von Gesamthämoglobin definiert ist,
   und mindestens einen Indikator von Blutcalciummetabolismus, wobei der mindestens eine Indikator von Blutcalciummetabolismus durch die Blutkonzentration von 25(OH)-Vitamin $D_3$ des Patienten definiert ist,

   b) Setzen der biologischen Parameter in Korrelation, dadurch Bereitstellen eines Mortalitätsrisikowertes, wobei der Risikowert indikativ für die Mortalität des Patienten innerhalb der nächsten 10 Jahre ist und wobei der Risikowert indikativ für eine Behandlung von sekundärer Vorbeugung des Patienten ist, das Mortalitätsrisiko des Patienten aufgrund der kardiovaskulären Erkrankung zu reduzieren, **dadurch gekennzeichnet, dass** der Mortalitätsrisikowert ein vereinfachter Risikowert $X_{1\#}$ ist und die biologischen Parameter in Korrelation durch eine Summe von mindestens acht Größen gesetzt werden, wodurch die Summe den vereinfachten Risikowert $X_{1\#}$ bereitstellt,
   wobei eine erste Größe gleich 2 ist, wenn das Alter des Patienten gleich oder mehr als 75 Jahre ist, und ansonsten gleich 0 ist,
   wobei eine zweite Größe gleich 1 ist, wenn die Konzentration von N-terminalem pro-B-Typ-natriuretischem Peptid (NT-proBNP) gleich oder mehr als 400 ng/L ist, gleich 2, wenn die Konzentration von N-terminalen pro-B-Typ-natriuretischen Peptid (NT-proBNP) gleich oder mehr als 2000 ng/L ist, und ansonsten gleich 0,
   wobei eine dritte Größe gleich 1 ist, wenn die Konzentration von glykiertem Hämoglobin HbA1c gleich oder mehr als 6,5 % für Patienten mit bekanntem Diabetes ist, und ansonsten gleich 0,
   wobei eine vierte Größe gleich 1 ist, wenn die Konzentration von Renin gleich oder mehr als 50 pg/mL ist, und ansonsten gleich 0,
   wobei eine fünfte Größe gleich 1 ist, wenn die Konzentration von Cystatin C gleich oder mehr als 1,2 mg/L ist, oder ansonsten gleich 0, wobei eine sechste Größe gleich 1 ist, wenn die Konzentration von 25(OH)-Vitamin $D_3$ gleich oder weniger als 10 ng/L ist, und ansonsten gleich 0,
   wobei eine siebte Größe gleich 1 ist, wenn die Herzfrequenz des Patienten gleich oder mehr als 75 Schläge pro Minute ist, und ansonsten gleich 0, und
   wobei eine achte Größe gleich 1 ist, wenn das Geschlecht des Patienten männlich ist, und ansonsten gleich 0,
   wobei ein vereinfachter Risikowert $X_{1\#}$ von
   gleich 0 oder gleich 1 ein geringes Mortalitätsrisiko anzeigt,
   gleich 2 oder gleich 3 ein moderates Mortalitätsrisiko anzeigt,

gleich oder mehr als 4 ein hohes Mortalitätsrisiko anzeigt,
wobei ein geringes Mortalitätsrisiko ein Mortalitätsrisiko von unter 20 % bedeutet, ein moderates Mortalitätsrisiko ein Mortalitätsrisiko zwischen 30 bis 40 % bedeutet und ein hohes Mortalitätsrisiko ein Mortalitätsrisiko von mindestens 50 % bedeutet.

2. Verfahren nach Anspruch 1, wobei der Satz von biologischen Parametern eine weitere Variable in Form eines Indikators von myokardialer Funktion oder myokardialer Leistung umfasst, wobei der Indikator von myokardialer Funktion oder myokardialer Leistung durch die linke ventrikuläre Ejektionsfraktion (LVEF) des Patienten in Volumen pro Volumen-% definiert ist und wobei die biologischen Parameter in Korrelation gesetzt sind durch eine Summe von mindestens neun Größen, wodurch die Summe den vereinfachten Risikowert $X_{1a\#}$ bereitstellt, bevorzugt wobei die neunte Größe durch die linke ventrikuläre Ejektionsfraktion (LVEF) des Patienten in Volumen pro Volumen-% definiert ist und wobei die neunte Größe ist
gleich 1, wenn die linke ventrikuläre Ejektionsfraktion (LVEF) moderat reduziert ist,
gleich 2, wenn die linke ventrikuläre Ejektionsfraktion stark reduziert ist, und ansonsten gleich 0,
wobei eine moderat reduzierte linke ventrikuläre Ejektionsfraktion (LVEF) durch einen Wert von 30 - 44 in Volumen pro Volumen-% definiert ist und wobei eine stark reduzierte linke ventrikuläre Ejektionsfraktion (LVEF) durch einen Wert von unter 30 in Volumen pro Volumen-% definiert ist,
wobei ein vereinfachter Risikowert $X_{1a\#}$ von
gleich 0 oder gleich 1 ein geringes Mortalitätsrisiko anzeigt,
gleich 2 oder gleich 3 ein moderates Mortalitätsrisiko anzeigt,
gleich oder mehr als 4 ein hohes Mortalitätsrisiko anzeigt,
wobei ein geringes Mortalitätsrisiko ein Mortalitätsrisiko von unter 20 % bedeutet, ein moderates Mortalitätsrisiko ein Mortalitätsrisiko zwischen 30 bis 40 % bedeutet und ein hohes Mortalitätsrisiko ein Mortalitätsrisiko von mindestens 50 % bedeutet.

3. Verfahren nach Anspruch 1, wobei der Risikowert ein gewichteter Risikowert $X_{2\#}$ ist und die biologischen Parameter in Korrelation unter Verwenden der folgenden Formel gesetzt werden, dadurch Bereitstellen des gewichteten Risikowerts $X_{2\#}$:

$$X_{2\#} = (\text{Variable } 1 - 4{,}151) * 2{,}69 + (\text{Variable } 2 - 1{,}848) * 1{,}32 + (\text{Variable } 3 - 4{,}221) * 0{,}633 + \text{Variable } 4 * 0{,}182 + (\text{Variable } 5 - 5{,}786) * 0{,}345 + (\text{Variable } 6) * -0{,}495 + (\text{Variable } 7 - 3{,}168) * 0{,}172 + (\text{Variable } 8 - 2{,}688) * -0{,}492 + (\text{Variable } 9 - 0{,}019) * 0{,}593,$$

wobei

a) Variable 1 durch den log-Wert des Alters des Patienten in Jahren definiert ist,
b) Variable 2 durch den log-Wert der Blutkonzentration von glykiertem Hämoglobin HbA1c des Patienten in % von Gesamthämoglobin definiert ist,
c) Variable 3 durch den log-Wert der Herzfrequenz des Patienten in Schlägen pro Minute definiert ist,
d) Variable 4 als gleich 1 definiert ist, wenn die linke ventrikuläre Ejektionsfraktion (LVEF) des Patienten moderat reduziert ist, gleich 2, wenn die linke ventrikuläre Ejektionsfraktion (LVEF) stark reduziert ist, und ansonsten gleich 0,
wobei eine moderat reduzierte linke ventrikuläre Ejektionsfraktion definiert ist durch einen Wert von 30 - 44 in Volumen pro Volumen-% und wobei eine stark reduzierte linke ventrikuläre Ejektionsfraktion definiert ist durch einen Wert von unter 30 in Volumen pro Volumen-% ,
e) Variable 5 durch den log-Wert der Blutkonzentration von N-terminalem pro-B-Typ-natriuretischem Peptid (NT-proBNP) des Patienten in ng/L definiert ist,
f) Variable 6 als gleich 1 definiert ist, wenn das Geschlecht des Patienten männlich ist, und gleich 0, wenn das Geschlecht des Patienten weiblich ist,
g) Variable 7 durch den log-Wert des Blutkonzentration von Renin des Patienten in pg/ml definiert ist,
h) Variable 8 durch den log-Wert der Blutkonzentration von 25(OH)-Vitamin $D_3$ des Patienten in ng/L definiert ist,
i) Variable 9 durch den log-Wert der Blutkonzentration von Cystatin C des Patienten in mg/L definiert ist,
bevorzugt wobei ein gewichteter Risikowert $X_{2\#}$
in dem Bereich von weniger als oder gleich 0 ein geringes Mortalitätsrisiko anzeigt,
in dem Bereich von 0 bis 1 ein mittleres Mortalitätsrisiko anzeigt, und

in dem Bereich von mehr als oder gleich 1 ein hohes Mortalitätsrisiko anzeigt,
wobei ein geringes Mortalitätsrisiko ein Mortalitätsrisiko von unter 20 % bedeutet, ein moderates Mortalitätsrisiko ein Mortalitätsrisiko zwischen 30 bis 40 % bedeutet und ein hohes Mortalitätsrisiko ein Mortalitätsrisiko von mindestens 50 % bedeutet.

4. In vitro-Verfahren zum Bestimmen des Mortalitätsrisiko in einem Patienten mit einer kardiovaskulären Erkrankung, wobei die kardiovaskuläre Erkrankung eine stabile koronare Arterienerkrankung ist, umfassend die Schritte von

a) Analysieren eines Satzes von biologischen Parametern, die von dem Patienten erhalten werden, wobei der Satz von biologischen Parametern mindestens die folgenden Variablen umfasst: das Alter des Patienten, einen Indikator von myokardialer Leistung, einen Indikator von Nierenleistung, die Herzfrequenz des Patienten, einen Indikator von Blutglukosemetabolismus,
b) Setzen der biologischen Parameter in Korrelation, dadurch Bereitstellen eines Mortalitätsrisikowertes, wobei der Risikowert indikativ für die Mortalität des Patienten innerhalb der nächsten 10 Jahre ist und wobei der Risikowert indikativ für eine Behandlung von sekundärer Vorbeugung des Patienten ist, das Mortalitätsrisiko des Patienten aufgrund der kardiovaskulären Erkrankung zu reduzieren, wobei

i) der Indikator von myokardialer Leistung durch die linke ventrikuläre Ejektionsfraktion des Patienten in Volumen pro Volumen-% definiert ist,
ii) der Indikator von Nierenleistung durch die Blutkonzentration von Serumkreatinin des Patienten definiert ist,
iii) der Indikator von Blutglukosemetabolismus durch die Blutkonzentration von glykiertem Hämoglobin HbA1c des Patienten in % von Gesamthämoglobin definiert ist, und
wobei der Mortalitätsrisikowert ein vereinfachter Risikowert $X_{1a}$ ist und wobei die biologischen Parameter in Korrelation durch eine Summe von mindestens sechs Größen gesetzt werden, wodurch die Summe den vereinfachten Risikowert $X_{1a}$ bereitstellt,
wobei eine erste Größe gleich 2 ist, wenn das Alter des Patienten gleich oder mehr als 75 Jahre ist, und ansonsten gleich 0,
wobei eine zweite Größe gleich 1 ist, wenn die linke ventrikuläre Ejektionsfraktion moderat reduziert ist, gleich 2, wenn die linke ventrikuläre Ejektionsfraktion stark reduziert ist und ansonsten gleich 0, wobei eine moderat reduzierte linke ventrikuläre Ejektionsfraktion durch einen Wert von 30 - 44 in Volumen pro Volumen-% definiert ist und wobei eine stark reduzierte linke ventrikuläre Ejektionsfraktion durch einen Wert von unter 30 in Volumen pro Volumen-% definiert ist,
wobei eine dritte Größe gleich 1 ist, wenn die Konzentration von glykiertem Hämoglobin HbA1c gleich oder mehr als 6,5 % für Patienten mit bekanntem Diabetes ist, und ansonsten gleich 0,
wobei eine vierte Größe gleich 1 ist, wenn die Serumkreatininkonzentration gleich oder mehr als 1,3 mg/dl ist, und ansonsten gleich 0, und
wobei eine fünfte Größe gleich 1 ist, wenn die Herzfrequenz des Patienten gleich oder mehr als 75 Schläge pro Minute ist, und ansonsten gleich 0,
wobei der Satz von biologischen Parametern weiterhin eine Variable in Form eines Indikators von Leberleistung umfasst, wobei der Indikator von Leberleistung durch die Blutkonzentration von Serumcholinesterase des Patienten definiert ist und wobei eine sechste Größe auf 1 gesetzt wird, wenn die Konzentration von Serumcholinesterase gleich oder weniger als 4,4 Einheiten/ml ist, und ansonsten gleich 0,
wobei ein vereinfachter Risikowert $X_{1a}$ von
gleich 0 ein geringes Mortalitätsrisiko anzeigt,
gleich 1 oder gleich 2 ein mittleres Mortalitätsrisiko anzeigt,
gleich oder mehr als 3 ein hohes Mortalitätsrisiko anzeigt,
wobei ein geringes Mortalitätsrisiko ein Mortalitätsrisiko von unter 20 % bedeutet, ein mittleres Mortalitätsrisiko ein Mortalitätsrisiko zwischen 30 bis 50 % bedeutet und ein hohes Mortalitätsrisiko ein Mortalitätsrisiko von mindestens 60 % bedeutet.

5. Verfahren nach Anspruch 4, wobei der Risikowert ein gewichteter Risikowert $X_2$ ist und die biologischen Parameter in Korrelation unter Verwenden der folgenden Formel gesetzt werden, dadurch Bereitstellen des gewichteten Risikowerts $X_2$:

$$X_2 = (\text{Variable 1} - 4,14) * 2,33 + (\text{Variable 2}) * 0,42 + (\text{Variable 3} - 1,73) * -1,05 + (\text{Variable 4} - 0,68) * 1,27 + (\text{Variable 5} - 4,21) * 1,47 + (\text{Variable 6} - 1,85) * 1,79,$$

wobei

a) Variable 1 durch den log-Wert des Alters des Patienten in Jahren definiert ist,

b) Variable 2 als gleich 1 definiert ist, wenn die linke ventrikuläre Ejektionsfraktion moderat reduziert ist, gleich 2, wenn die linke ventrikuläre Ejektionsfraktion stark reduziert ist und ansonsten gleich 0, wobei eine moderat reduzierte linke ventrikuläre Ejektionsfraktion definiert ist durch einen Wert von 30 - 44 in Volumen pro Volumen-% und wobei eine stark reduzierte linke ventrikuläre Ejektionsfraktion definiert ist durch einen Wert von unter 30 in Volumen pro Volumen-%,

c) Variable 3 durch den log-Wert der Blutkonzentration von Serumcholinesterase des Patienten in Einheiten/ml definiert ist,

d) Variable 4 durch den log-Wert der Blutkonzentration von Serumkreatinin des Patienten in mg/dl definiert ist,

e) Variable 5 durch den log-Wert der Herzfrequenz des Patienten in Schlägen pro Minute definiert ist,

f) Variable 6 durch den log-Wert der Blutkonzentration von glykiertem Hämoglobin HbA1c des Patienten in % von Gesamthämoglobin definiert ist,

bevorzugt wobei ein gewichteter Risikowert $X_2$ in dem Bereich von weniger als oder gleich 0 ein geringes Mortalitätsrisiko anzeigt,

in dem Bereich von 0 bis 1 ein mittleres Mortalitätsrisiko anzeigt, und

in dem Bereich von mehr als oder gleich 1 ein hohes Mortalitätsrisiko anzeigt,

wobei ein geringes Mortalitätsrisiko ein Mortalitätsrisiko von unter 20 % bedeutet, ein moderates Mortalitätsrisiko ein Mortalitätsrisiko zwischen 30 bis 40 % bedeutet und ein hohes Mortalitätsrisiko ein Mortalitätsrisiko von mindestens 50 % bedeutet.

6. Verfahren nach beliebigen der vorhergehenden Ansprüche, wobei die Behandlung von sekundärer Vorbeugung in Form von verabreichten Wirkstoffen ist, wobei die Wirkstoffen zum Behandeln von Hyperlipidämie, Regulieren von Blutdruck, Modifizieren der Herzfrequenz und / oder zum Bereitstellen von glykämischer Kontrolle bei Diabetespatienten sind, bevorzugt wobei die Wirkstoffe zum Reduzieren der Blutkonzentration von Cholesterin des Patienten, zum Reduzieren des Blutdrucks des Patienten und / oder zum Reduzieren der Blutglukosekonzentration des Patienten sind.

7. Verfahren nach beliebigen der vorhergehenden Ansprüche, wobei der Risikowert für eine Behandlung von sekundärer Vorbeugung von Patienten mit einem mittleren oder moderaten Mortalitätsrisiko oder von Patienten mit einem hohen Mortalitätsrisiko indikativ ist.

8. Computerprogrammprodukt, das direkt in den internen Speicher eines Digitalcomputers ladbar ist, umfassend Softwarecodeteile zum Implementieren der Berechnung des Mortalitätsrisikowertes gemäß einem beliebigen der Ansprüche 1 bis 5, wenn dieses Produkt auf einem Computer gefahren wird, bevorzugt umfassend Softwarecodeteile zum Implementieren der Berechnung des gewichteten Risikowerts wie in Anspruch 3 oder Anspruch 5 definiert.

**Revendications**

1. Procédé in vitro pour déterminer le risque de mortalité chez un patient atteint d'une maladie cardio-vasculaire, dans lequel la maladie cardiovasculaire est une maladie coronarienne stable, comprenant les étapes consistant à

a) analyser un ensemble de paramètres biologiques obtenus du patient, où l'ensemble de paramètres biologiques comprend les variables suivantes :

le sexe du patient,
l'âge du patient,
au moins un indicateur de fonction myocardique ou de performance myocardique,
dans lequel l'au moins un indicateur de fonction myocardique ou de performance myocardique est défini par la fraction d'éjection du ventricule gauche (FEVG) en % du volume par volume du patient, ou par la concentration sanguine de propeptide N-terminal du peptide natriurétique de type B (NT-proBNP) du patient, au moins deux indicateurs de fonction rénale ou de performance rénale, où les au moins deux indicateurs de fonction rénale ou de performance rénale sont définis par la concentration sanguine de rénine et cystatine C du patient,

la fréquence cardiaque du patient,

au moins un indicateur de métabolisme de la glycémie, où l'au moins un indicateur de métabolisme de la glycémie est défini par la concentration sanguine d'hémoglobine glyquée HbAlc en % de l'hémoglobine totale du patient,

et au moins un indicateur de métabolisme du calcium dans le sang, où l'au moins un indicateur de métabolisme du calcium dans le sang est défini par la concentration sanguine de 25(OH)-vitamine $D_3$ du patient,

b) mettre les paramètres biologiques en corrélation, fournissant ainsi un score de risque de mortalité, où le score de risque est indicatif pour la mortalité du patient au cours des 10 prochaines années, et où le score de risque est indicatif pour un traitement de prévention secondaire du patient pour réduire le risque de mortalité du patient imputable à la maladie cardio-vasculaire, **caractérisé en ce que**

le score de risque de mortalité est un score de risque simplifié $X_{1\#}$ et les paramètres biologiques sont mis en corrélation par une somme d'au moins huit grandeurs, la somme fournissant ainsi le score de risque simplifié $X_{1\#}$,

dans lequel une première grandeur est égale à 2 si l'âge du patient est supérieur ou égal à 75 ans et sinon égale à 0,

dans lequel une deuxième grandeur est égale à 1 si la concentration de propeptide N-terminal du peptide natriurétique de type B (NT-proBNP) est supérieure ou égale à 400 ng/L, égale à 2 si la concentration de propeptide N-terminal du peptide natriurétique de type B (NT-proBNP) est supérieure ou égale à 2000 ng/L et sinon égale à 0,

dans lequel une troisième grandeur est égale à 1 si la concentration d'hémoglobine glyquée HbAlc est supérieure ou égale à 6,5 % pour les patients atteints de diabète connu et sinon égale à 0,

dans lequel une quatrième grandeur est égale à 1 si la concentration de rénine est supérieure ou égale à 50 pg/ml et sinon égale à 0,

dans lequel une cinquième grandeur est égale à 1 si la concentration de cystatine C est supérieure ou égale à 1,2 mg/L ou sinon égale à 0,

dans lequel une sixième grandeur est égale à 1 si la concentration de 25(OH)-vitamine $D_3$ est inférieure ou égale à 10 ng/L et sinon égale à 0,

dans lequel une septième grandeur est égale à 1 si la fréquence cardiaque du patient est supérieure ou égale à 75 battements par minute et sinon égale à 0, et

dans lequel une huitième grandeur est égale à 1 si le sexe du patient est masculin et sinon égale à 0,

dans lequel un score de risque simplifié $X_{1\#}$

égal à 0 ou égal à 1 indique un faible risque de mortalité,

égal à 2 ou égal à 3 indique un risque de mortalité modéré,

supérieur ou égal à 4 indique un risque de mortalité élevé,

dans lequel un faible risque de mortalité signifie un risque de mortalité inférieur à 20 %, un risque de mortalité modéré signifie un risque de mortalité entre 30 et 40 %, et un risque de mortalité élevé signifie un risque de mortalité d'au moins 50 %.

2. Procédé selon la revendication 1, dans lequel l'ensemble de paramètres biologiques comprend une variable supplémentaire sous la forme d'un indicateur de fonction myocardique ou de performance myocardique, dans lequel l'indicateur de fonction myocardique ou de performance myocardique est défini par la fraction d'éjection du ventricule gauche (FEVG) en % du volume par volume du patient, et dans lequel les paramètres biologiques sont mis en corrélation par une somme d'au moins neuf grandeurs, la somme fournissant ainsi le score de risque simplifié $X_{1a\#}$, de préférence

dans lequel la neuvième grandeur est définie par la fraction d'éjection du ventricule gauche (FEVG) en % du volume par volume du patient et dans lequel la neuvième grandeur est

égale à 1 si la fraction d'éjection du ventricule gauche (FEVG) est modérément réduite,

égale à 2 si la fraction d'éjection du ventricule gauche est fortement réduite et sinon égale à 0,

dans lequel une fraction d'éjection du ventricule gauche (FEVG) modérément réduite est définie par une valeur de 30 à 44 en % du volume par volume, et dans lequel une fraction d'éjection du ventricule gauche (FEVG) fortement réduite est définie par une valeur inférieure à 30 en % de volume par volume,

dans lequel un score de risque simplifié $X_{1a\#}$

égal à 0 ou égal à 1 indique un faible risque de mortalité,

égal à 2 ou égal à 3 indique un risque de mortalité modéré,

supérieur ou égal à 4 indique un risque de mortalité élevé,
dans lequel un faible risque de mortalité signifie un risque de mortalité inférieur à 20 %, un risque de mortalité modéré signifie un risque de mortalité entre 30 et 40 %, et un risque de mortalité élevé signifie un risque de mortalité d'au moins 50 %.

**3.** Procédé selon la revendication 1, dans lequel le score de risque est un score de risque pondéré $X_{2\#}$ et les paramètres biologiques sont mis en corrélation en utilisant la formule suivante, fournissant ainsi le score de risque pondéré $X_{2\#}$ :

$$X_{2\#} = (\text{variable } 1 - 4,151) * 2,69 + (\text{variable } 2 - 1,848) * 1,32 + (\text{variable } 3 - 4,221) * 0,633 + \text{variable } 4 * 0,182 + (\text{variable } 5 - 5,786) * 0,345 + (\text{variable } 6) * -0,495 + (\text{variable } 7 - 3,168) * 0,172 + (\text{variable } 8 - 2,688) * -0,492 + (\text{variable } 9 - 0,019) * 0,593,$$

dans lequel

a) la variable 1 est définie par la valeur logarithmique de l'âge en années du patient,
b) la variable 2 est définie par la valeur logarithmique de la concentration sanguine d'hémoglobine glyquée HbAlc en % de l'hémoglobine totale du patient,
c) la variable 3 est définie par la valeur logarithmique de la fréquence cardiaque en battements par minute du patient,
d) la variable 4 est définie comme étant égale à 1 si la fraction d'éjection du ventricule gauche (FEVG) du patient est modérément réduite, égale à 2 si la fraction d'éjection du ventricule gauche (FEVG) est fortement réduite et sinon égale à 0,
dans lequel une fraction d'éjection du ventricule gauche modérément réduite est définie par une valeur de 30 à 44 en % du volume par volume, et dans lequel une fraction d'éjection du ventricule gauche fortement réduite est définie par une valeur inférieure à 30 en % de volume par volume,
e) la variable 5 est définie par la valeur logarithmique de la concentration sanguine de propeptide N-terminal du peptide natriurétique de type B (NT-proBNP) en ng/L du patient,
f) la variable 6 est définie comme étant égale à 1 si le sexe du patient est masculin et égale à 0 si le sexe du patient est féminin,
g) la variable 7 est définie par la valeur logarithmique de la concentration sanguine de rénine en pg/ml du patient,
h) la variable 8 est définie par la valeur logarithmique de la concentration sanguine de 25(OH)-vitamine $D_3$ en ng/L du patient,
i) la variable 9 est définie par la valeur logarithmique de la concentration sanguine de cystatine C en mg/L du patient,
de préférence dans lequel un score de risque pondéré $X_{2\#}$
dans la plage inférieure ou égale à 0 indique un faible risque de mortalité,
dans la plage de 0 à 1 indique un risque de mortalité intermédiaire, et
dans la plage de supérieure ou égale à 1 indique un risque de mortalité élevé,
dans lequel un faible risque de mortalité signifie un risque de mortalité inférieur à 20 %, un risque de mortalité modéré signifie un risque de mortalité entre 30 et 40 %, et un risque de mortalité élevé signifie un risque de mortalité d'au moins 50 %.

**4.** Procédé in vitro pour déterminer le risque de mortalité chez un patient atteint d'une maladie cardio-vasculaire, dans lequel la maladie cardiovasculaire est une maladie coronarienne stable, comprenant les étapes consistant à

a) analyser un ensemble de paramètres biologiques obtenus du patient, où l'ensemble de paramètres biologiques comprend au moins les variables suivantes : l'âge du patient, un indicateur de performance myocardique, un indicateur de performance rénale, la fréquence cardiaque du patient, un indicateur de métabolisme de la glycémie,
b) mettre les paramètres biologiques en corrélation, fournissant ainsi un score de risque de mortalité, où le score de risque est indicatif pour la mortalité du patient au cours des 10 prochaines années, et où le score de risque est indicatif pour un traitement de prévention secondaire du patient pour réduire le risque de mortalité d'un patient imputable à la maladie cardiovasculaire,

dans lequel

i) l'indicateur de performance myocardique est défini par la fraction d'éjection du ventricule gauche en % du volume par volume du patient,

ii) l'indicateur de performance rénale est défini par la concentration sanguine de créatinine sérique du patient,

iii) l'indicateur de métabolisme de la glycémie est défini par la concentration sanguine d'hémoglobine glyquée HbAlc en % de l'hémoglobine totale du patient, et

dans lequel le score de risque de mortalité est un score de risque simplifié $X_{1a}$ et dans lequel les paramètres biologiques sont mis en corrélation par une somme d'au moins six grandeurs, la somme fournissant ainsi le score de risque simplifié $X_{1a}$,

dans lequel une première grandeur est égale à 2 si l'âge du patient est supérieur ou égale à 75 ans et sinon égale à 0,

dans lequel une deuxième grandeur est égale à 1 si la fraction d'éjection du ventricule gauche est modérément réduite, égale à 2 si la fraction d'éjection du ventricule gauche est fortement réduite et sinon égale à 0, dans lequel une fraction d'éjection du ventricule gauche modérément réduite est définie par une valeur de 30 à 44 en % du volume par volume, et dans lequel une fraction d'éjection du ventricule gauche fortement réduite est définie par une valeur inférieure à 30 en % du volume par volume,

dans lequel une troisième grandeur est égale à 1 si la concentration d'hémoglobine glyquée HbAlc est supérieure ou égale à 6,5 % pour les patients atteints de diabète connu et sinon égale à 0,

dans lequel une quatrième grandeur est égale à 1 si la concentration de créatinine sérique est supérieure ou égale à 1,3 mg/dl et sinon égale à 0, et

dans lequel une cinquième grandeur est égale à 1 si la fréquence cardiaque du patient est supérieure ou égale à 75 battements par minute et sinon égale à 0,

dans lequel l'ensemble de paramètres biologiques comprend en outre une variable sous la forme d'un indicateur de performance hépatique, dans lequel l'indicateur de performance hépatique est défini par la concentration sanguine de cholinestérase sérique du patient et dans lequel une sixième grandeur est mise à 1 si la concentration de cholinestérase sérique est inférieure ou égale à 4,4 unités/ml et sinon égale à 0,

dans lequel un score de risque simplifié $X_{1a}$ égal à 0 indique un faible risque de mortalité,

égal à 1 ou égal à 2 indique un risque de mortalité intermédiaire,

supérieur ou égal à 3 indique un risque de mortalité élevé,

dans lequel un faible risque de mortalité signifie un risque de mortalité inférieur à 20 %, un risque de mortalité intermédiaire signifie un risque de mortalité entre 30 et 50 %, et un risque de mortalité élevé signifie un risque de mortalité d'au moins 60 %.

5. Procédé selon la revendication 4, dans lequel le score de risque est un score de risque pondéré $X_2$ et les paramètres biologiques sont mis en corrélation en utilisant la formule suivante, fournissant ainsi le score de risque pondéré $X_2$ :

```
X₂ = (variable 1 - 4,14) * 2,33 + (variable 2) * 0,42 +
(variable 3 - 1,73) * -1,05 + (variable 4 - 0,68) * 1,27 +
(variable 5 - 4,21) * 1,47 + (variable 6 - 1,85) * 1,79,
```

dans lequel

a) la variable 1 est définie par la valeur logarithmique de l'âge en années du patient,

b) la variable 2 est définie comme étant égale à 1 si la fraction d'éjection du ventricule gauche est modérément réduite, égale à 2 si la fraction d'éjection du ventricule gauche est fortement réduite et sinon égale à 0, dans lequel une fraction d'éjection du ventricule gauche modérément réduite est définie par une valeur de 30 à 44 en % du volume par volume, et dans lequel une fraction d'éjection du ventricule gauche fortement réduite est définie par une valeur inférieure à 30 en % du volume par volume,

c) la variable 3 est définie par la valeur logarithmique de la concentration sanguine de cholinestérase sérique en unités/ml du patient,

d) la variable 4 est définie par la valeur logarithmique de la concentration sanguine de créatinine sérique en mg/dl du patient,

e) la variable 5 est définie par la valeur logarithmique de la fréquence cardiaque en battements par minute du patient,

f) la variable 6 est définie par la valeur logarithmique de la concentration sanguine d'hémoglobine glyquée HbAlc en % de l'hémoglobine totale du patient,
de préférence dans lequel un score de risque pondéré $X_2$ dans la plage inférieure ou égale à 0 indique un faible risque de mortalité,
dans la plage de 0 à 1 indique un risque de mortalité intermédiaire, et
dans la plage de supérieure ou égale à 1 indique un risque de mortalité élevé,
dans lequel un faible risque de mortalité signifie un risque de mortalité inférieur à 20 %, un risque de mortalité modéré signifie un risque de mortalité entre 30 et 40 %, et un risque de mortalité élevé signifie un risque de mortalité d'au moins 50 %.

6.  Procédé selon l'une quelconque des revendications précédentes, dans lequel le traitement de prévention secondaire est sous la forme de médicaments administrés, dans lequel les médicaments sont destinés à traiter l'hyperlipidémie, à réguler la pression sanguine, à modifier la fréquence cardiaque et/ou à fournir un contrôle glycémique chez des patients diabétiques, de préférence dans lequel les médicaments sont destinés à réduire la concentration sanguine de cholestérol du patient, à réduire la pression sanguine du patient, et/ou à réduire la glycémie du patient.

7.  Procédé selon l'une quelconque des revendications précédentes, dans lequel le score de risque est indicatif pour un traitement de prévention secondaire de patients présentant un risque de mortalité intermédiaire ou modéré, ou de patients présentant un risque de mortalité élevé.

8.  Produit de programme informatique pouvant être chargé directement dans la mémoire interne d'un ordinateur numérique, comprenant des parties de code logiciel pour mettre en œuvre le calcul du score de risque de mortalité selon l'une quelconque des revendications 1 à 5 lorsque ledit produit est exécuté sur un ordinateur, de préférence comprenant des parties de code logiciel pour mettre en œuvre le calcul du score de risque pondéré tel que défini dans la revendication 3 ou la revendication 5.

**Figure 1**

Figure 2

# Figure 3

# Figure 4

## Figure 5

# Figure 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FORD, ES et al.** *N. Engl. J. Med.,* 2007, vol. 356, 2388-2398
- **IX et al.** *Circulation,* 2010, vol. 121, 1295-1303
- **STENVINKEL et al.** *Clin J Am Soc Nephrol,* 2008, vol. 3, 505-521
- **SCHNABEL et al.** *European Heart Journal,* 2010, vol. 31, 3024-3031
- **THOMAS et al.** *Diabetes Care,* 2012, vol. 35, 1158-1164
- **DOWDY ; WEARDEN.** Statistics for Research. John Wiley & Sons, 1983
- **SCHNABEL et al.** *Eur Heart J,* 2010, vol. 31, 3024-3031
- **BONOW.** *Circulation,* 1996, vol. 93, 1946
- **SMITH.** *J Endocrinol,* 2000, vol. 167, 239
- **MUELLER.** *Clin Chem Lab Med,* 2004, vol. 42, 942
- **WU.** *Clin Chem,* 2004, vol. 50, 867
- **ALA-KOPSALA.** *Clin Chem,* 2004, vol. 50, 1576
- **SCHNABEL et al.** *Eur Heart J,* 2010, vol. 31, 2024-3031
- ICSI Health Care Guidelines. 2011
- **MARSCHNER et al.** *J Am College Cardiol,* 2001, vol. 38, 56-63
- **GOLIASCH et al.** *Eur Heart J,* 2012
- **GOLIASCH et al.** *Eur Heart J,* 2012, vol. 33, 2282-2289
- **BASSAND J.P et al.** *Eur Heart J,* 2007, vol. 28, 1598-1660
- **FUSTER V. et al.** *N Engl J Med,* 1992, vol. 326, 242-250
- **MORROW D.A.** *Circulation,* 2010, vol. 121, 2681-2691
- **STEG P.G. et al.** *JAMA,* 2007, vol. 297, 1197-1206
- **GRAHAM I. et al.** *Eur Heart J,* 2007, vol. 28, 2375-2414
- **CLARK A.M et al.** *Ann Intern Med,* 2005, vol. 143, 659-672
- **MARSCHNER I.C. et al.** *J Am Coll Cardiol,* 2001, vol. 38, 56-63
- **MANJUNATH G. et al.** *J Am Coll Cardiol,* 2003, vol. 41, 47-55
- **SACHDEV M. et al.** *J Am Coll Cardiol,* 2004, vol. 43, 576-582
- **NIESSNER A. et al.** *Thromb Haemost,* 2005, vol. 93, 949-954
- **WINKELMANN B.R. et al.** *Pharmacogenomics,* 2001, vol. 2, S1-73
- **TOMASCHITZ A. et al.** *Eur Heart J,* 2010, vol. 31, 1237-1247
- **PENCINA M.J. et al.** *Stat Med,* 2008, vol. 27, 157-172
- **GRANGER C.B. et al.** *Arch Intern Med,* 2003, vol. 163, 2345-2353
- **HEMINGWAY H. et al.** *BMJ,* 2009, vol. 339, b4184
- **YAN A.T. et al.** *Eur Heart J,* 2007, vol. 28, 1072-1078
- **LIBBY P.** *Circulation,* 1995, vol. 91, 2844-2850
- **NAGHAVI M. et al.** *Circulation,* 2003, vol. 108, 1664-1672
- **BOGATY P. et al.** *Circulation,* 1993, vol. 87, 1938-1946
- **VRANCKX P. et al.** *EuroIntervention,* 2011, vol. 7, 859-871
- **SCHNABEL R.B. et al.** *Eur Heart J,* 2010, vol. 31, 3024-3031
- **EMOND M. et al.** *Circulation,* 1994, vol. 90, 2645-2657
- **MOCK M.B. et al.** *Circulation,* 1982, vol. 66, 562-568
- **CALDERON-MARGALIT R. et al.** *Clin Chem,* 2006, vol. 52, 845-852
- **GOLIASCH G. et al.** *Clin Chem,* 31 January 2012
- **WANG T.J.** *J Am Coll Cardiol,* 2010, vol. 55, 2092-2095
- **KOOTER A.J. et al.** *Circulation,* 2011, vol. 124, 741-745
- **WILSON P.W.** *Am J Med,* 2012, vol. 125, 695-703
- **OMLAND T.** *Circulation,* 2002, vol. 106, 2913-8
- **DE LEMOS J.A.** *N Engl J Med,* 2001, vol. 345, 1014-21
- **WANG T.J.** *N Engl J Med,* 2006, vol. 355, 2631-9
- **PARIKH N.I.** *Eur Heart J,* 2007, vol. 28, 2644-52
- **TOMASCHITZ A.** *Eur Heart J,* 2011, vol. 32, 2642-9
- **THOMAS G.N.** *Diabetes Care,* 2012, vol. 35, 1158-64